# EUROPEAN PATENT SPECIFICATION

(11) **EP 4 267 078 B1**
(45) Date of publication and mention of the grant of the patent: **10.12.2025**
(21) Application number: 21843730.9
(22) Date of filing: 22.12.2021
(51) Int. Cl.: A61K 8/02, A61K 8/19, A61K 8/24, A61K 8/34, A61K 8/365, A61Q 15/00

(54) **PROCESS FOR TREATING HUMAN PERSPIRATION AND BODY ODORS USING MAGNESIUM OXIDE AND A PHOSPHATE SALT**
VERFAHREN ZUR BEHANDLUNG VON SCHWEISS UND KÖRPERGERÜCHEN UNTER VERWENDUNG VON MAGNESIUMOXID UND EINES PHOSPHATSALZES
PROCÉDÉ DE TRAITEMENT DE LA TRANSPIRATION HUMAINE ET DES ODEURS CORPORELLES À L'AIDE DE MAGNÉSIE ET D'UN SEL DE PHOSPHATE

(30) Priority: 22.12.2020 FR 2013937
(43) Date of publication of application: 01.11.2023
(73) Proprietor: L'OREAL, 75008 Paris (FR)
(72) Inventor: AJILI, Widad, 94152 CHEVILLY-LARUE (FR); GALEY, Jean-Baptiste, 93601 AULNAY-SOUS-BOIS (FR)
(74) Representative: Lavoix
(86) International application number: PCT/EP2021/087210
(87) International publication number: WO 2022/136507

(56) References cited:
- WO-A2-2013/013902
- JP-A- 2004 154 253
- US-A1- 2020 276 091

## Description

This invention relates to a cosmetic process for treating human perspiration and treating body odors resulting from perspiration, which comprises the use of magnesium oxide and at least one water-soluble phosphate acid salt.

The underarms as well as certain other parts of the body are generally the place of several discomforts that can stem directly or not from perspiration problems. These phenomena often cause unpleasant and uncomfortable sensations which are primarily due to the presence of sweat resulting from perspiration that can, in certain cases, make the skin moist and wet clothing, in particular at the underarms or the back, as such leaving visible traces. Moreover, the presence of sweat can generate the discharging of body odors that are most of the time unpleasant. Finally, during the evaporation thereof, sweat can also leave behind salts and/or proteins on the surface of the skin which can cause white traces on clothing. Such discomforts are to be taken into account including in the case of moderate perspiration.

In the cosmetics field, it is well known to use as topical application, antiperspirant products containing substances that have the effect of limiting and even suppressing the sweat flow so as to remedy the problems mentioned hereinabove. These products are in general available in the form of roll-on, sticks, aerosol or spray.

The antiperspirant substances generally consist of aluminum and/or zirconium hydrochlorides. These substances make it possible to reduce the sweat flow by forming a plug on the sweat duct. Furthermore, they have a deodorant effect thanks to the antibacterial properties thereof.

However, consumers are increasingly seeking antiperspirant products containing no, or very little, aluminum and/or zirconium hydrochlorides.

Furthermore, these substances can also leave traces when they are applied which has the consequence of staining clothing.

In order to overcome all of these disadvantages mentioned hereinabove and address the requirement, it has been proposed to seek other effective active substances, that are well tolerated by the skin and easy to formulate.

As an alternative to aluminum and/or zirconium hydrochlorides, in the application WO2019/072831, a combination between a cation Xⁿ⁺ of valency n, an anion Y^{m-} of valency m and a modulating agent has previously been proposed. The cation is particularly alkaline-earth such as magnesium chloride, and the anion is particularly an alkali metal salt. However, such compositions have no deodorant effect, but merely antiperspirant properties. Furthermore, the use of chloride ions in large quantities (for example in the form of MgCl₂ of the order of 20% by weight) can give rise to corrosion problems during the manufacture of the composition. In particular, this requires specific equipment, different for example from conventional stainless steel tanks.

Therefore, there is a real need to have compositions having both antiperspirant and deodorant properties, which do not have all of the drawbacks described above, i.e., which provide both an antiperspirant effect (particularly in terms of effectiveness and resistance to sweat) and a deodorant effect, which are well tolerated by the skin, and which can be manufactured conventionally (i.e., without using specific industrial equipment). Finally, such compositions must be capable of being formulated in a way that is stable in storage.

The Applicant discovered surprisingly that this objective could be achieved using a cosmetic process for treating human perspiration and treating body odors resulting from perspiration, which uses magnesium oxide and at least one water-soluble phosphate acid salt. Indeed, by reacting the magnesium present in magnesium oxide, and the phosphate of a water-soluble phosphate acid salt (such as XH₂PO₄), a magnesium phosphate precipitate is obtained, which can obstruct the entrance of the sweat pore, particularly based on the following reaction:

MgO + XH₂PO₄ → MgXPO₄ + H₂O

Such a mixture has both antiperspirant and deodorant properties in contact with sweat. Indeed, when sweat appears on the surface of skin treated with magnesium oxide and the water-soluble phosphate acid salt, said phosphate salt is solubilized in the sweat, which enables the above reaction to take place. On the other hand, while the mixture remains anhydrous, no reaction takes place.

Such a mixture is furthermore devoid of chloride, and therefore does not require specific, in particular corrosion-proof, industrial equipment.

This invention therefore relates to a cosmetic process for treating human perspiration and treating body odors resulting from perspiration, comprising the use of magnesium oxide and at least one water-soluble phosphate acid salt that is potassium dihydrogen phosphate, the molar ratio between the water-soluble phosphate acid salt and magnesium oxide being strictly less than 1.

Surprisingly, unlike magnesium chloride, magnesium oxide MgO is very slightly soluble in water, but it is capable of reacting with a phosphate acid salt solubilized in water. Furthermore, in this way, the antiperspirant effect is only triggered in contact with an aqueous medium such as sweat: the effect observed according to the invention is therefore an antiperspirant effect triggered "on demand".

When magnesium oxide and the water-soluble phosphate acid salt are formulated in an anhydrous medium, they can be present within the same composition (i.e., a single composition). Such an anhydrous composition can further comprise at least one modulating agent.

Alternatively, they can be formulated in two different compositions, one comprising magnesium oxide (composition A), and the other comprising the water-soluble phosphate acid salt (composition B). An optional modulating agent can be present in composition A and/or in composition B.

When magnesium oxide and/or the water-soluble phosphate acid salt are formulated in an aqueous medium (i.e., comprising an aqueous phase, for example an aqueous solution or an oil-in-water or water-in-oil solution), they are preferably formulated in two different compositions, one comprising magnesium oxide (composition A), and the other comprising the water-soluble phosphate acid salt (composition B). An optional modulating agent can be present in composition A and/or in composition B.

Preferably, this invention relates to a cosmetic process for treating human perspiration and treating body odors resulting from perspiration, which comprises:
(i) either the mixing just before use of at least one composition A and of at least one composition B, said compositions A and B being packaged separately, followed by the application of the resulting mixture on the surface of the skin;
(ii) or the application on the surface of the skin simultaneously or sequentially of at least one composition A and of at least one composition B packaged separately;
(iii) or the application on the surface of the skin of a composition comprising in the same holder at least one composition A and of at least one composition B;
(iv) or the application on the surface of the skin of a composition, preferably anhydrous, comprising magnesium oxide and a water-soluble phosphate acid salt that is potassium dihydrogen phosphate, provided that said magnesium oxide and said salt are not in contact with one another in said composition.

Obviously, composition A comprises in a cosmetically acceptable medium magnesium oxide, and composition B comprises in a cosmetically acceptable medium at least one water-soluble phosphate acid salt that is potassium dihydrogen phosphate.

Composition A and/or in composition B can further comprise at least one modulating agent.

Such a process according to the invention is effective in the treatment of human perspiration. Furthermore, compositions A and B are stable in storage.

This invention also relates to a ready-to-use cosmetic composition, particularly for treating human perspiration and treating body odors resulting from perspiration, which comprises, in a cosmetically acceptable medium, magnesium oxide and at least one water-soluble phosphate acid salt that is potassium dihydrogen phosphate, the molar ratio between the water-soluble phosphate acid salt and magnesium oxide being strictly less than 1. This composition is referred to as "ready-to-use composition" in this application. This ready-to-use composition can further comprise at least one modulating agent.

"Ready-to-use" means that either (a) the composition is applied on the surface of the skin within a very short interval following the preparation thereof, for example from a few seconds to a few minutes following the preparation thereof; or (b) the composition comprises magnesium oxide and a water-soluble phosphate acid salt that is potassium dihydrogen phosphate, and said magnesium oxide and said salt are not in contact with one another in said composition. Typically according to (a), the time between the mixing of magnesium oxide with at least one water-soluble phosphate acid salt, optionally with at least one modulator, and the application on the skin, is from 0 to 30 minutes, preferably from 0 to 10 minutes, preferably from 0 to 1 minute, and preferably from 0 to 30 seconds.

"Antiperspirant agent" or "antiperspirant active agent" means any substance or any composition that has the effect of reducing sweat flow and/or reducing the damp sensation on the skin associated with human sweat and/or masking human sweat.

"Cosmetic process for treating human perspiration" means a process which, used on human skin, reduces sweat flow and/or the damp sensation associated with human sweat.

"Deodorant agent" or "deodorant active agent" means any substance or any composition capable of substantially reducing, or even eliminating, unpleasant odors, in particular body odor, in particular unpleasant odor resulting from the decomposition of human sweat by bacteria.

"Cosmetic process for treating body odors resulting from perspiration" means a process which, used on human skin, substantially reduces, or even eliminates, unpleasant odors, in particular body odor, in particular unpleasant odor resulting from the decomposition of human sweat by bacteria.

"Cosmetically acceptable medium" means a medium compatible with the skin and/or appendages or mucosa thereof, having a pleasant color, odor and texture and not giving rise to discomfort (such as tightness), liable to dissuade the consumer from using the composition.

Said cosmetically acceptable medium is also a medium that does not leave any traces during the application thereof, and that thus does not stain clothing.

Said cosmetically acceptable medium can be anhydrous or comprise an aqueous phase.

"Sequential" means a successive administration.

"Same holder" means that compositions A and B according to the invention are present in the same packaging, in particular in a two-compartment packaging that allows for the simultaneous application of compositions A and B.

"Composition comprising magnesium oxide and a water-soluble phosphate acid salt, provided that said magnesium oxide and said salt are not in contact with one another in said composition" means a composition wherein each agent (magnesium oxide and water-soluble phosphate acid salt) remains as is and does not interact with one another. In such a composition, no chemical reaction takes place between magnesium oxide and said salt.

According to a first embodiment, the composition comprising magnesium oxide and a water-soluble phosphate acid salt, provided that said magnesium oxide and said salt are not in contact with one another in said composition, is an anhydrous composition.

According to a second embodiment, the composition comprising magnesium oxide and a water-soluble phosphate acid salt, provided that said magnesium oxide and said salt are not in contact with one another in said composition is a composition comprising an aqueous phase wherein each agent is present in a separate phase and does not interact with one another. This is for example the case of a composition in multiple emulsion form where the two agents are present in two separate agents are present in two separate aqueous phases separated by an oily phase which would preventing them meeting.

"Anhydrous" means a composition comprising less than 1% by weight of water relative to the total weight of the composition, preferably less than 0.5% by weight, preferably less than 0.1% by weight. Preferably, an anhydrous composition is a composition totally free from water.

### Magnesium oxide

"Magnesium oxide" means the compound of formula MgO.

Preferably, composition A comprises magnesium oxide at a content between 1 and 20% by weight relative to the total weight of composition A, preferably between 2 and 10% by weight.

Likewise, preferably, the single anhydrous composition or the ready-to-use composition comprises magnesium oxide at a content between 1 and 20% by weight relative to the total weight of the composition, preferably between 2 and 10% by weight.

According to a specific form of the invention, composition A comprises magnesium oxide, and contains no water-soluble phosphate acid salt.

According to another form of the invention, the "ready-to-use composition" comprises magnesium oxide and at least one water-soluble phosphate acid salt.

### Water-soluble phosphate acid salt

"Water-soluble phosphate acid salt" means a water-soluble salt of mono- (HPO₄²⁻) or dihydrogen phosphate (H₂PO₄⁻), associated with a cation. The cation can particularly be an alkali cation.

In terms of this invention, the term "water-soluble salt" means any salt that, after having been completely dissolved with stirring at 0.5% in a solution of water at a temperature of 25°C, leads to a solution comprising a quantity of insoluble salt less than 0.05% by weight. The water-soluble phosphate acid salt is potassium dihydrogen phosphate (KH₂PO₄).

Preferably, composition B comprises at least one water-soluble phosphate acid salt at a content between 1 and 30% by weight relative to the total weight of composition B, preferably between 2 and 20% by weight.

Likewise, preferably, the single anhydrous composition or the ready-to-use composition comprises at least one water-soluble phosphate acid salt at a content between 1 and 30% by weight relative to the total weight of the composition, preferably between 2 and 20% by weight.

According to a specific form of the invention, composition B comprises at least one water-soluble phosphate acid salt, and contains no magnesium oxide.

According to another form of the invention, the "ready-to-use composition" comprises magnesium oxide and at least one water-soluble phosphate acid salt that is potassium dihydrogen phosphate.

According to the invention, the molar ratio between the water-soluble phosphate acid salt(s) and magnesium oxide (i.e., water-soluble phosphate acid salt/magnesium oxide molar ratio, KH₂PO₄/MgO molar ratio) is strictly less than 1, preferably less than or equal to 0.9, preferably less than or equal to 0.8, preferably less than or equal to 0.5. Preferably, this molar ratio is between 0.2 and 0.9, preferably between 0.3 and 0.8.

### Modulators

As stated above, the modulating agent(s) can be present in composition A and/or in composition B.

"Modulating agent" means a substance or a composition capable of modulating the precipitation of magnesium phosphate.

The modulating agent according to the invention is different from magnesium oxide and the water-soluble phosphate acid salt.

Preferably, it can be chosen from:
- mono- or polycarboxylic acids (preferably di- or tricarboxylic), optionally hydroxylated (i.e., hydroxyacids), in free or salified form, such as citric acid, propionic acid, tartaric acid, lactic acid, malic acid, succinic acid, glutaric acid, or itaconic acid,
- amino carboxylic acids in free or salified form such as aspartic acid, glutamic acid, serine, alanine, dehydroalanine and the oligomers thereof, iminosuccinic acid and the derivatives thereof, or ethylene diamine tetraacetic acid,
- monosaccharides, oligosaccharides, polysaccharides and the derivatives thereof. Preferably, the monosaccharides are chosen from glucose, galactose, mannose, xylose, lyxose, fucose, arabinose, rhamnose, ribose, deoxyribose, quinovose, fructose, sorbose, talose, threose and erythrose. Preferably, the oligosaccharides comprise from 2 to 6 monosaccharide units, and are preferably chosen from trehalose, lactose, maltose and cellobiose. The derivatives are preferably chosen from glucuronic and lactobionic acid. Finally, the polysaccharides are preferably chosen from alginates, chitosans and pectins,
- ascorbic acid,
- phytic acid,
- polymers or copolymers of carboxylic acids in free or salified form such as the products sold under the name SOKOLAN CP42, CP44 by BASF,
- polymers or copolymers of amino carboxylic acids in free or salified form such as polyaspartic acid such as for example the polymers mentioned in patents WO 9216462, WO 9403527 Srchem Incorp. and in particular a solution of sodium polyaspartate at 30% in water such as the product sold under the trade name AQUADEW SPA-30 by Ajinomoto; polyglutamic acid,
- polymers or copolymers of maleic or itaconic acid, and
- polymers or copolymers of carboxymethylinulin.

Among the preferential modulators, where they must be added, citric acid, ascorbic acid, lactic acid, propionic acid, tartaric acid or polyaspartic acid, in free or salified form, or a polymer or copolymer of carboxylic acids optionally amino will be used more particularly.

The modulating agent(s) according to the invention can be present in composition A and/or B (or in the ready-to-use composition or in the single anhydrous composition) at a content between 0.01 and 30% by weight relative to the total weight of composition A and/or B (or of the ready-to-use composition or of the single anhydrous composition), preferably between 1 and 10% by weight.

### Method of application

In order to obtain an antiperspirant effect and a deodorant effect on the skin, according to a first alternative embodiment of the process according to the invention (embodiment (i)), composition A comprising magnesium oxide and composition B comprising at least one water-soluble phosphate acid salt that is potassium dihydrogen phosphate are packaged separately and are mixed just before use (extemporaneous mixture), then the mixture thus obtained is applied on the surface of the skin to be treated.

According to a second alternative embodiment of the antiperspirant process according to the invention (embodiment (ii)), composition A comprising magnesium oxide and composition B comprising at least one water-soluble phosphate acid salt that is potassium dihydrogen phosphate packaged separately and applied simultaneously or sequentially on the surface of the skin to be treated.

According to this alternative embodiment, when compositions A and B are applied sequentially, the time interval separating the application of composition A from application B can vary from 1 second to 24 hours, more preferably from 10 seconds to 24 hours and even more preferably from 1 minute to 1 hour.

In order to obtain an antiperspirant effect and a deodorant effect on the skin, according to a third alternative embodiment of the process according to the invention (embodiment (iii)), a composition comprising in the same holder composition A containing magnesium oxide, composition B containing at least one water-soluble phosphate acid salt that is potassium dihydrogen phosphate and optionally at least one modulator is applied directly on the surface of the skin.

According to a fourth alternative embodiment of the process according to the invention (embodiment (iv)), which is a preferred alternative embodiment, a composition comprising, in a cosmetically acceptable medium, magnesium oxide, at least one water-soluble phosphate acid salt that is potassium dihydrogen phosphate and optionally at least one modulator, provided that said magnesium oxide and said salt are not in contact with one another in said composition, is applied directly on the surface of the skin.

Compositions A and B can, each one independently, be anhydrous or comprise an aqueous phase. When they are aqueous, they can be in the form of aqueous solutions, alcoholic or hydroalcoholic solutions; in the form of emulsions (oil-in-water or water-in-oil); in the form of aqueous gels; or in the form of aqueous dispersions.

With regards in particular to the aforementioned embodiment (iii), compositions A and B can be for example packaged in a device comprising at least two compartments containing respectively composition A and composition B such as a twin-tube, a pump bottle with two compartments, an aerosol device comprising two compartments that can include one or more outlet orifices (single-nozzle or double-nozzle), a device provided with a perforated wall such as a grid comprising two compartments; a device comprising two compartments each provided with a ball applicator (multi-ball roll-on); a double-stick.

Preferably, the product according to the fourth alternative embodiment of the process according to the invention (embodiment (iv)), in the form of anhydrous composition comprising, in a cosmetically acceptable medium, at least one water-soluble phosphate acid salt that is potassium dihydrogen phosphate and optionally at least one modulator, will be applied.

### Dosage forms

Compositions A and/or B can independently of one another be presented in any of the dosage forms conventionally used for topical application and particularly in the form of aqueous gels, aqueous solutions or hydroalcoholic solutions. Compositions A and/or B or the ready-to-use composition can also be anhydrous. They may also, by the addition of an oily or oil phase, be in the form of dispersions such as lotion, emulsions of liquid or semiliquid consistency such as milk, obtained by dispersing an oily phase in an aqueous phase (O/W) or conversely (W/O), or suspensions or emulsions of soft, semi-solid or solid consistency such as cream or gel, or multiple emulsions (W/O/W or O/W/O), microemulsions, ionic and/or non-ionic type vesicle dispersions, or wax/aqueous phase dispersions. These compositions A and/or B (or the ready-to-use composition or the single anhydrous composition) are prepared according to the usual methods.

Compositions A and/or B may be particularly packaged in pressurized form in an aerosol device or in a pump bottle; packaged in a device equipped with a perforated wall particularly a grid; packaged in a device equipped with a roll-on applicator; packaged in stick form, in loose or compacted powder form. In this respect, they contain the ingredients generally used in this type of products and well-known to a person skilled in the art.

According to another specific form of the invention, compositions A and/or B or the ready-to-use composition or the single anhydrous composition, can be solid in particular in the form of a stick; in loose or compacted powder form or in the form of dispersion in an anhydrous solvent such as an oil.

"Solid composition" means that the measurement of the maximum force measured by means of texturometric analysis on inserting a probe in the sample of formula should be at least equal to 0.25 Newton, in particular at least equal to 0.30 Newton, particularly at least equal to 0.35 Newton, assessed under precise measurement conditions as follows.

To take texturometric measurements, the formulas are poured when heated into jars 4 cm in diameter and 3 cm at the bottom. Cooling is performed at ambient temperature. The hardness of the formulas produced is measured after waiting 24 hours. The jars containing the samples are characterized by means of texturometric analysis using a texturometer such as that marketed by Rheo TA-XT2, according to the following protocol: a 5 mm diameter stainless steel ball type probe is brought into contact with the sample at a speed of 1 mm/s. The measurement system detects the interface with the sample with a detection threshold equal to 0.005 Newton. The probe is inserted 0.3 mm into the sample, at a rate of 0.1 mm/s. The measurement apparatus records the progression of the compression force measured over time, during the penetration phase. The hardness of the sample corresponds to the mean of the maximum values of the force detected during penetration, for at least 3 measurements.

The invention also relates to a cosmetic process for treating human perspiration, and treating body odors associated with human perspiration, comprising application on the surface of the skin, in particular on the surface of the underarms, of an effective quantity of composition A and an effective quantity of composition B; or an effective quantity of the ready-to-use composition; or an effective quantity of the single anhydrous composition.

The application time of composition A and/or B (or of the ready-to-use composition or of the single anhydrous composition) on the surface of the skin can vary from 0.5 to 10 seconds, preferably from 1 to 5 seconds.

These compositions A and B (or the ready-to-use composition or the single anhydrous composition) can be applied several times on the surface of the skin. They can be applied several times, over one day or over several days.

Another object of this invention is an aerosol device formed by a first receptacle comprising a pressurized composition A, a second receptacle comprising a pressurized composition B and by a dispensing means of the mixture of said composition.

The dispensing means, forming part of the aerosol device, generally consist of a dispensing valve controlled by a dispensing head, in turn comprising a nozzle via which pressurized compositions A and B are sprayed as a mixture. The receptacle containing pressurized compositions A and B can be opaque or transparent. It can be made of glass, polymeric or metal material, optionally coated with a layer of protective varnish.

Another object of this invention is an aerosol device formed by a receptacle comprising a pressurized anhydrous composition comprising, in a cosmetically acceptable medium, magnesium oxide, at least one water-soluble phosphate acid salt and optionally at least one modulating agent; and by a means for dispensing the mixture of said composition.

The dispensing means, forming part of the aerosol device, generally consist of a dispensing valve controlled by a dispensing head, in turn comprising a nozzle via which the pressurized anhydrous composition is sprayed. The receptacle containing the pressurized anhydrous composition can be opaque or transparent. It can be made of glass, polymeric or metal material, optionally coated with a layer of protective varnish.

Compositions A and/or B (or the ready-to-use composition or the single anhydrous composition) can also comprise at least one additional deodorant agent and/or at least one additional antiperspirant agent.

By way of illustration of additional deodorant agents, mention can be made in particular of bacteriostatic agents or bactericidal agents acting on germs of underarm odor, such as 2,4,4'-trichloro-2'-hydroxydiphenylether (Triclosan^{®}), 2,4-dichloro-2'-hydroxydiphenylether, 3',4',5'-trichlorosalicylanilide,1-(3',4'-dichlorophenyl)-3-(4'-chlorophenyl)urea (Triclocarban^{®}) or 3,7,11-trimethyldodeca-2,5,10-trienol (Farnesol^{®}); quaternary ammonium salts such as cetyltrimethylammonium salts, cetylpyridinium salts; polyols such as those of the glycerin type, 1,3-propanediol (ZEMEA PROPANEDIOL^{®} sold by Dupont Tate and Lyle Bioproducts), 1,2-decanediol (Symclariol^{®} from Symrise); glycerin derivatives such as for example Caprylic/Capric Glycerides (CAPMUL MCM^{®} from Abitec), Caprylate or glyceryl caprylate (DERMOSOFT GMCY^{®} and DERMOSOFT GMC^{®} respectively from STRAETMANS), Polyglyceryl-2 Caprate (DERMOSOFT DGMC^{®} from STRAETMANS), biguanide derivatives such as polyhexamethylene biguanide salts; chlorhexidine and salts thereof; 4-Phenyl-4,4-dimethyl-2butanol (SYMDEO MPP^{®} from Symrise); cyclodextrins; or alum.

The additional deodorant agents can be present preferably in the compositions according to the invention in mass concentrations ranging from 0.01% to 10% by weight relative to the total weight of the composition.

By way of illustration of additional antiperspirant agents, mention can be made in particular of aluminum and/or zirconium antiperspirant salts or complexes, preferably chosen from aluminum halohydrates; aluminum and zirconium halohydrates, zirconium hydroxychloride and aluminum hydroxychloride complexes with or without an amino acid such as those described in patent US-3792068.

Among the aluminum salts, mention can be made in particular of aluminum chlorohydrate in activated or non-activated form, aluminum chlorohydrex, aluminum chlorohydrex polyethyleneglycol complex, aluminum chlorohydrex propyleneglycol complex, aluminum dichlorohydrate, aluminum dichlorohydrex polyethyleneglycol complex, aluminum dichlorohydrex propyleneglycol complex, aluminum sesquichlorohydrate, aluminum sesquichlorohydrex polyethylene glycol complex, aluminum sesquichlorohydrex propyleneglycol complex, aluminum sulfate buffered by aluminum and sodium lactate.

Among the aluminum and zirconium salts, mention can be made in particular of aluminum zirconium octachlorohydrate, aluminum zirconium pentachlorohydrate, aluminum zirconium tetrachlorohydrate, aluminum zirconium trichlorohydrate.

Zirconium hydroxychloride and aluminum hydroxychloride complexes with an amino acid are generally known under the name ZAG (when the amino acid is glycine). Among these products, mention can be made of complexes of aluminum zirconium octachlorohydrex glycine, aluminum zirconium pentachlorohydrex glycine, aluminum zirconium tetrachlorohydrex glycine and aluminum zirconium trichlorohydrex glycine.

Aluminum sesquichlorohydrate is in particular sold under the trade name REACH 301^{®} by SUMMITREHEIS.

Among the aluminum and zirconium salts, mention can be made of the complexes of zirconium hydroxychloride and aluminum hydroxychloride with an amino acid such as glycine having for INCI name: ALUMINUM ZIRCONIUM TETRACHLOROHYDREX GLY for example that sold under the trade name REACH AZP-908-SUF^{®} by SUMMITREHEIS.

Use will be made more particularly of aluminum chlorohydrate in optionally activated form sold under the trade names LOCRON S FLA^{®}, LOCRON P, LOCRON L.ZA by CLARIANT; under the trade names MICRODRY ALUMINUM CHLOROHYDRATE^{®}, MICRO-DRY 323^{®}, CHLORHYDROL 50, REACH 103, REACH 501 by SUMMITREHEIS; under the trade name WESTCHLOR 200^{®} by WESTWOOD; under the trade name ALOXICOLL PF 40^{®} by GUILINI CHEMIE; CLURON 50%^{®} by Industria Quimica Del Centro; CHLOROHIDROXIDO ALUMINIO SO A 50%^{®} by Finquimica.

As another antiperspirant agent, mention can be made of the particles of expanded perlite such as those obtained by the method of expansion described in the patent US 5,002,698.

Preferably, compositions A and/or B (or the ready-to-use composition or the single anhydrous composition) comprise less than 5% by weight of aluminum salt, preferably less than 3% by weight, preferably less than 1% by weight.

Preferably, compositions A and/or B (or the ready-to-use composition or the single anhydrous composition) are totally free of aluminum salt.

### Aqueous phase

Compositions A and/or B (or the ready-to-use composition) can comprise at least one aqueous phase. They are formulated particularly in aqueous lotions or in a water-in-oil emulsion, an oil-in-water emulsion, or in a multiple emulsion (triple oil-in-water-in-oil or water-in-oil-in-water emulsion) (such emulsions are known and described for example by C. FOX in "Cosmetics and Toiletries" - November 1986 - Vol 101 - pages 101-112).

The aqueous phase of said compositions A and/or B (or the ready-to-use composition) contains water and generally other solvents soluble in water or miscible with water. The water-soluble or miscible solvents comprise short-chain mono-alcohols for example C₁-C₄ such as ethanol, isopropanol; diols or polyols such as ethyleneglycol, 1,2-propyleneglycol, 1,3-butylene glycol, hexyleneglycol, diethyleneglycol, dipropylene glycol, 2-ethoxyethanol, diethylene glycol monomethylether, triethylene glycol monomethylether and sorbitol. Propyleneglycol and glycerin, propane 1,3 diol shall more particularly be used.

### Emulsifiers

### a) Oil-in-water emulsifiers

Compositions A and/or B (or the ready-to-use composition) can comprise at least one surfactant.

The surfactants can be of any sort usually used in cosmetics, such as anionic surfactants, cationic surfactants, amphoteric surfactants or non-ionic surfactants.

Preferably, non-ionic surfactants are used such as:
- C8-C30 (preferably C12-C18) polyoxyethylenated fatty alcohols, having in particular from 2 to 100 moles of ethylene oxide, such as oxyethylene ether of cetearyl alcohol with 30 oxyethylene groups (CTFA name "Ceteareth-30"), oxyethylene ether of stearyl alcohol with 20 oxyethylene groups (CTFA name "Steareth-20") such as BRIJ 78 marketed by UNIQEMA, or oxyethylene ether of cetearyl alcohol with 33 oxyethylene groups (CTFA name "Ceteareth-33");
- C8-C30 fatty alcohol and sugar, in particular alkyl (C8-C30) (poly)glucosides, alone or in mixtures with alcohols, such as the mixture of cetylstearyl alcohol and of cocoglucoside sold under the name MONTANOV 82^{®} by Seppic, the mixture of arachidyl alcohol and of behenyl alcohol with arachidylglucoside sold under the name MONTANOV 802^{®} by Seppic, the mixture of myristyl alcohol and of myristylglucoside sold under the name MONTANOV 14^{®} by Seppic, the mixture of cetylstearyl alcohol and of cetylstearylglucoside sold under the name MONTANOV 68^{®} by Seppic, the mixture of C14-C22 alcohol with C12-C20 alkylglucoside sold under the name MONTANOV L^{®} by Seppic, the mixture of cocoalcohol and cocoglucoside sold under the name MONTANOV S^{®} by Seppic or the mixture of isostearyl alcohol and isostearylglucoside sold under the name MONTANOV WO 18^{®} by Seppic;
- ethers of polyethylene glycol, having in particular from 20 to 120 ethylene oxide units, and esters of C8-C30 fatty acids and glucose or methylglucose,
- esters of C8-C30 fatty acid and sorbitan,
- esters of C8-C30 fatty acids and polyoxyethylenated sorbitan, having in particular from 2 to 30 moles of ethylene oxide,
- esters of C8-C30 polyoxyethylenated fatty acids and sorbitan, having in particular from 2 to 100 moles of ethylene oxide,
- mono or diesters of C8-C30 fatty acids and glycerol,
- esters of C8-C30 polyglycerolated fatty acids, having in particular from 2 to 16 moles of glycerol,
- esters of C8-C30 fatty acids and polyethylene glycol, having in particular from 2 to 200 ethylene oxide units,
- esters of C8-C30 fatty acids and glucose or of alkyl(C1-C2)glucose or sucrose, and
- mixtures thereof.

The surfactant(s) can be present in a quantity ranging from 0.1 to 10% by weight, relative to the total weight of composition A or B (or of the ready-to-use composition), preferably ranging from 0.2 to 5% by weight, and preferably ranging from 1% to 4% by weight.

### b) Water-in-oil emulsifiers

Among the emulsifiers that can be used in the water-in-oil emulsions or triple water-in-oil-in-water-in-oil emulsions or triple emulsions, mention can be made as an example of alkyl dimethicone copolyols having the following formula (I) 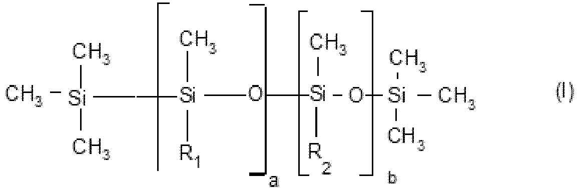 wherein:
R₁ designates a C₁₂ to C₂₀ linear or branched alkyl group and preferably C₁₂ to C₁₈;
R₂ designates the group: -CₙH₂ₙ-(-OC₂H₄-)ₓ-(-OC₃H₆-)_{y}-O-R₃,
R₃ represents a hydrogen atom or a linear or branched alkyl radical having from 1 to 12 carbon atoms;
a is an integer varying from 1 to about 500;
b designates an integer varying from 1 to about 500;
n is an integer varying from 2 to 12 and preferably from 2 to 5;
x designates an integer varying from 1 to about 50 and preferably from 1 to 30;
y designates an integer varying from 0 to about 49 and preferably 0 to 29 provided that when y is different from zero the ratio x/y is greater than 1 and preferably varies from 2 to 11.

Among the preferred copolyol alkyldimethicone emulsifiers having formula (I), mention will be made particularly of CETYL PEG/PPG-10/1 DIMETHICONE and more particularly the mixture CETYL PEG/PPG-10/1 DIMETICONE AND DIMETHICONE (INCI name) such as the product sold under the trade name ABIL EM90 by GOLDSCHMIDT, LAURYL PEG/PPG-18/18 methicone and more particularly the mixture LAURYL PEG/PPG-18/18 methicone and DODECENE and POLOXAMER 407 such as the product sold under the trade name DOW CORNING 5200 FORMULATION AID by DOW CORNING or the mixture (Polyglyceryl-4-stearate and Cetyl PEG/PPG-10 (and) Dimethicone (and) Hexyl Laurate) such as the product sold under the trade name ABIL WE09 by GOLDSCHMIDT.

Among the water-in-oil emulsifiers, mention can also be made of copolyol dimethicones having the following formula (II) 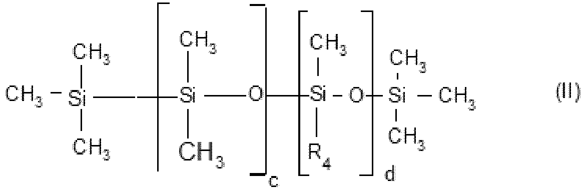 wherein
R₄ designates the group -CₘH₂ₘ-(-OC₂H₄-)ₛ-(-OC₃H₆-)ₜ-O-R₅,
R₅ designates a hydrogen atom or a linear or branched alkyl radical having from 1 to 12 carbon atoms;
c is an integer varying from 1 to about 500,
d designates an integer varying from 1 to about 500,
n is an integer varying from 2 to 12 and preferably from 2 to 5,
s designates an integer varying from 1 to about 50, and preferably from 1 to 30;
t designates an integer varying from 0 to about 50 and preferably from 0 to 30; provided that the sum s+t is greater than or equal to 1.

Among these preferred copolyol dimethicone emulsifiers having formula (II), use will be made in particular of PEG-18/PPG-18 DIMETHICONE and more particularly the mixture CYCLOPENTASILOXANE (and) PEG-18/PPG-18 DIMETHICONE (INCI name) such as the product sold by Dow Corning under the trade name Silicone DC 5225 C or KF-6040 by Shin Etsu.

According to a particularly preferred embodiment, a mixture of at least one emulsifier having formula (I) and of at least one emulsifier having formula (II) will be used.

Use will more particularly be made of a mixture of PEG-18/PPG-18 Dimethicone and Cetyl PEG/PPG-10/1 DIMETHICONE, LAURYL PEG/PPG-18/18 methicone and even more particularly a mixture of (CYCLOPENTASILOXANE (and) PEG-18/PPG-18 Dimethicone) and of Cetyl PEG/PPG-10/1 DIMETICONE and Dimethicone or of (Polyglyceryl-4-stearate and Cetyl PEG/PPG-10 (and) Dimethicone (and) Hexyl Laurate) or the mixture LAURYL PEG/PPG-18/18 methicone and DODECENE and POLOXAMER 407.

Among the water-in-oil emulsifiers, mention can also be made of the non-ionic emulsifiers derived from fatty acids and from polyol, alkylpolyglycosides (APG), esters of sugars and the mixtures thereof.

As non-ionic emulsifiers derived from fatty acids and from polyol, it is possible to use in particular fatty acid esters and polyol, the fatty acid in particular having a C8-C24 alkyl chain, and the polyols being for example glycerol and sorbitan.

As esters of fatty acid and polyol, mention can be made in particular of esters of isostearic acid and polyols, esters of stearic acid and polyols, and mixtures thereof, in particular esters of isostearic acid and glycerol and/or sorbitan.

As esters of stearic acid and polyols, mention can be made in particular of polyethyleneglycol esters such as PEG-30 Dipolyhydroxystearate such as the product marketed under the name Arlacel P135 by ICI.

The esters of glycerol and/or sorbitan include, for example, polyglycerol isostearate, such as the product marketed under the name Isolan GI 34 by Goldschmidt; sorbitan isostearate, such as the product marketed under the name Arlacel 987 by ICI; sorbitan isostearate and glycerol, such as the product marketed under the name Arlacel 986 by ICI, the mixture of sorbitan isostearate and of polyglycerol isostearate (3 moles) marketed under the name Arlacel 1690 by Unigema and mixtures thereof.

The emulsifier can also be chosen from alkylpolyglycosides having an HLB less than 7, for example those represented by the following general formula: R-O-(G)x
wherein R represents a branched and/or unsaturated alkyl radical, comprising from 14 to 24 carbon atoms, G represents a reduced sugar comprising from 5 to 6 carbon atoms, and x designates a value ranging from 1 to 10 and preferably from 1 to 4, and G in particular designates glucose, fructose or galactose.

The unsaturated alkyl radical can comprise one or more ethylene unsaturations, and in particular one or two ethylene unsaturations.

As alkylpolyglycosides of this type, mention can be made of alkylpolyglucosides (G=glucose in the formula above), and in particular the compounds having formula (I) wherein R represents more particularly an oleyl radical (C18 unsaturated radical) or isostearyl (C18 saturated radical), G designates glucose, x is a value varying from 1 to 2, in particular isostearyl-glucoside, oleyl-glucoside and mixtures thereof. This alkylpolyglucoside can be used in a mixture with a co-emulsifier, more especially with a fatty alcohol and in particular a fatty alcohol having the same fatty chain as that of alkylpolyglucoside, i.e., comprising from 14 to 24 carbon atoms and having a branched and/or unsaturated chain, and for example isostearyl alcohol when the alkylpolyglucoside is isostearyl-glucoside, and the oleyl alcohol when the alkylpolyglucoside is oleyl-glucoside, optionally in the form of a self-emulsifying composition, such as described for example in document WO-A-92/06778. It is possible to use for example the mixture of isostearyl-glucoside and of isostearyl alcohol, marketed under the name Montanov WO 18 by SEPPIC as well as the mixture octyldodecanol and octyldodecylxyloside marketed under the name FLUDANOV 20X by SEPPIC.

Mention can also be made of polyolefins with a succinic termination, such as polyisobutylenes with an esterified succinic termination and the salts thereof, in particular the diethanolamine salts, such as the products marketed under the names Lubrizol 2724, Lubrizol 2722 and Lubrizol 5603 by Lubrizol or the commercial product CHEMCINNATE 2000.

The total quantity in emulsifier(s) in the composition A or B (or in the ready-to-use composition) shall preferably have active substance contents varying from 1 to 8% by weight and more particularly from 2 to 6% by weight relative to the total weight of the composition.

According to a specific form of the invention, compositions A and/or B (or in the ready-to-use composition) in the form of an emulsion can be prepared according to the phase inversion manufacturing technique. This technique is, in its principle, well known and in particular described in the article "Phase Inversion Emulsification", by Th Förster et al, published in Cosmetics & Toiletries, vol. 106, December 1991, pp 49-52. Its principle is as follows:
(i) In the presence of a suitable emulsifying system, with stirring, an oily phase on the one hand and an aqueous phase on the other hand are mixed, said mixture being carried out at a temperature greater than the phase inversion temperature (PIT) of the medium, in such a way as to obtain an emulsion of the water-in-oil type.
(ii) The temperature of the emulsion thus obtained is brought to a temperature less than said phase inversion temperature, whereby an ultrafine emulsion of the oil-in-water type is obtained.
(iii) Mineral nanopigments are then introduced during the implementation of the step (i) and/or at the end of the step (ii).

The suitable systems are emulsifiers of the non-ionic type and are chosen from polyoxyethylenated and/or polyoxypropylenated fatty alcohols (i.e., compounds obtained by a reaction between an aliphatic fatty alcohol, such as behenyl alcohol or cetyl alcohol, with ethylene oxide or propylene oxide or an ethylene oxide/propylene oxide mixture) and the esters of fatty acids and of polyols, optionally polyoxyethylenated and/or polyoxypropylenated (i.e., compounds obtained by a reaction of a fatty acid, such as stearic acid or oleic acid, with a polyol, such as for example an alkyleneglycol or glycerol or a polyglycerol, optionally in the presence of ethylene oxide or propylene oxide or an ethylene oxide/propylene oxide mixture), or mixtures thereof.

Moreover, and preferably, the emulsifying system retained will have a global HLB (HLB = Hydrophilic-Lipophilic Balance, in terms of Griffin; see J. Soc. Cosm. Chem. 1954 (vol 5), pp 249-256; balance between the hydrophilic nature and the lipophilic nature of the surfactant) ranging from 9.5 to about 11.5, advantageously close to 10, in such a way as to make it possible to obtain a phase inversion at a temperature less than 90°C (TIP<90°C).

The content of emulsifying agent(s) is between 0.5 and 40% by weight and preferably between 2 and 10% by weight relative to the total weight of the emulsion.

### Oily phase

The compositions A and/or B (or the ready-to-use composition or the single anhydrous composition) can contain at least one non-water-miscible organic liquid phase, known as a fatty phase. This generally includes one or a plurality of hydrophobic compounds rendering said phase non-miscible in water. Said phase is liquid (in the absence of a structuring agent) at ambient temperature (20-25°C). Preferentially, the organic liquid phase non-miscible in water according to the invention is generally comprised of at least one volatile oil and/or one non-volatile oil and optionally at least one structuring agent.

"Oil" means a fat that is liquid at ambient temperature (25°C) and atmospheric pressure (760mm Hg namely 10⁵ Pa). The oil may be volatile or non-volatile.

The term "volatile oil" according to the invention denotes any oil capable of evaporating in contact with skin or keratin fiber in less than one hour, at ambient temperature and atmospheric pressure. The volatile oils according to the invention are volatile cosmetic oils that are liquid at ambient temperature, having a vapor pressure different to zero, at ambient temperature and atmospheric pressure, particularly ranging from 0.13 Pa to 40,000 Pa (10⁻³ to 300 mm Hg), particularly ranging from 1.3 Pa to 13,000 Pa (0.01 to 100 mm Hg), and more specifically ranging from 1.3 Pa to 1300 Pa (0.01 to 10 mm Hg).

The term "non-volatile oil" denotes an oil remaining on skin or keratin fiber at ambient temperature and atmospheric pressure for at least several hours and particularly having a vapor pressure less than 10⁻³ mm Hg (0.13 Pa).

The oil may be chosen from any physiologically acceptable and particularly cosmetically acceptable oils, in particular mineral, animal, plant, synthetic oils; in particular, volatile or non-volatile hydrocarbon and/or silicone and/or fluorinated oils and mixtures thereof.

More specifically, the term "hydrocarbon oil" denotes an oil essentially comprising carbon and hydrogen atoms and optionally one or a plurality of functions chosen from hydroxyl, ester, ether, carboxylic functions. Generally, the oil has a viscosity of 0.5 to 100,000 mPa.s, preferably from 50 to 50,000 mPa.s and more preferably from 100 to 300,000 mPa.s.

By way of examples of volatile oils suitable for use in the invention, mention can be made of:
- volatile hydrocarbon oils chosen from hydrocarbon oils having 8 to 16 carbon atoms, and particularly petroleum-based C₈-C₁₆ isoalkanes (also referred to as isoparaffins) such as isododecane (also referred to as 2,2,4,4,6-pentamethylheptane), isodecane, isohexadecane, and for example the oils sold under the trade names Isopars or Permetyls, C₈-C₁₆ branched esters, iso-hexyl neopentanoate, and mixtures thereof. Further volatile hydrocarbon oils such as petroleum distillates, particularly those sold under the name Shell Solt by SHELL, can also be used; volatile linear alkanes such as those described in patent application of Cognis DE10 2008 012 457.

- volatile silicones, such as for example volatile linear or cyclic silicone oils, particularly those having a viscosity less than or equal to 8 centistokes (8 10⁻⁶ m²/s), and having in particular 2 to 7 silicon atoms, these silicones optionally comprising alkyl or alkoxy groups having 1 to 10 carbon atoms. Mention can be made, as a volatile silicone oil suitable for use in the invention, in particular, of octamethyl cyclotetrasiloxane, decamethyl cyclopentasiloxane, dodecamethyl cyclohexasiloxane, heptamethyl hexyltrisiloxane, heptamethyloctyl trisiloxane, hexamethyl disiloxane, octamethyl trisiloxane, decamethyl tetrasiloxane, dodecamethyl pentasiloxane;
- and mixtures thereof.

Mention can also be made of volatile alkyl trisiloxane linear oils with general formula (III): 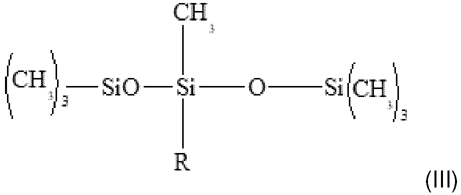 where R represents an alkyl group comprising 2 to 4 carbon atoms and in which one or more hydrogen atoms can be substituted by a fluorine or chlorine atom.

Among oils with general formula (III), mention can be made of:
3-butyl 1,1,1,3,5,5,5-heptamethyl trisiloxane,
3-propyl 1,1,1,3,5,5,5-heptamethyl trisiloxane, and
3-ethyl 1,1,1,3,5,5,5-heptamethyl trisiloxane,
corresponding to the oils having formula (III) for which R is respectively a butyl group, a propyl group or an ethyl group.

By way of examples of non-volatile oils suitable for use in the invention, mention can be made of:
- plant-based hydrocarbon oils such as liquid fatty acid triglycerides having 4 to 24 carbon atoms such as heptanoic or octanoic triglycerides or wheat germ, olive oils, sweet almond, palm, rapeseed, cotton, coconut, alfalfa, poppy seed, pumpkin, squash, blackcurrant seed, evening primrose, millet, barley, quinoa, rye, safflower, candlenut, passiflora, musk rose, sunflower, corn, soybean, squash, grape seed, sesame, hazelnut, apricot, macadamia, castor, avocado oils, caprylic/capric acid triglycerides such as those sold by Stearineries Dubois or those sold under the trade names Miglyol 810, 812 and 818 by SASOL, jojoba oil, shea butter;

- linear or branched hydrocarbons of mineral or synthetic origin, such as liquid paraffins and derivatives thereof, petroleum jelly, polydecenes, polybutenes, hydrogenated polyisobutene such as Parleam, squalane;
- synthetic ethers having from 10 to 40 carbon atoms;
- synthetic esters particular of fatty acids such as the oils having the formula R₁COOR₂ wherein R₁ represents the residue of a linear or branched higher fatty acid comprising 1 to 40 carbon atoms and R₂ represents a hydrocarbon chain, particularly branched containing 1 to 40 carbon atoms with R₁ + R₂ ≥ 10 such as for example Purcellin oil (cetostearyl octanoate)), isononyl isononanoate, isopropyl myristate, isopropyl palmitate, C₁₂ to C₁₅ alcohol benzoate, hexyl laurate, diisopropyl adipate, isononyl isononanoate, 2-ethylhexyl palmitate, octyl-2-dodecyl stearate, octyl-2-dodecyl erucate, isostearyl isostearate, tridecyl trimellitate; octanoates, decanoates or ricinoleates of alcohols or polyalcohols such as propylene glycol dioctanoate; hydroxylated esters, such as isostearyl lactate, octyl hydroxy stearate, octyl dodecyl hydroxy stearate, diisostearyl-malate, triisocetyl citrate; heptanoates, octanoates, decanoates of fatty alcohols; polyol esters, such as propylene glycol dioctanoate, neopentyl glycol diheptanoate and diethylene glycol diisononanoate; and pentaerythritol esters such as pentaerythrityl tetraisostearate;
- fatty alcohols that are liquid at ambient temperature, with a branched and/or unsaturated carbon chain having 12 to 26 carbon atoms, such as octyldodecanol, isostearyl alcohol, 2-butyloctanol, 2-hexyl decanol, 2-undecylpentadecanol, oleic alcohol;
- higher fatty acids, such as oleic acid, linoleic acid, linolenic acid;
- fluorinated oils optionally partially hydrocarbon-based and/or silicone-based, such as fluorosilicone oils, fluorinated polyethers or fluorinated silicones, as described in document EP-A-847 752;
   silicone oils, such as polydimethylsiloxanes (PDMS) which are non-volatile and linear or cyclic; polydimethylsiloxanes comprising alkyl, alkoxy or phenyl groups which are pendant or at the end of the silicone chain, said groups having from 2 to 24 carbon atoms; phenylated silicones, such as phenyl trimethicones, phenyl dimethicones, phenyltrimethylsiloxydiphenylsiloxanes, diphenyl dimethicones, diphenylmethyldiphenyl-trisiloxanes or (2-phenylethyl)trimethylsiloxysilicates; and
- mixtures thereof.

Compositions A and/or B (or the ready-to-use composition or the single anhydrous composition) can comprise at least one solid fatty acid chosen preferably from waxes and pasty fats, and mixtures thereof and more particularly waxes.

### Pasty fats

For the purposes of the invention, the term "pasty fat" denotes a lipophilic fatty compound having a reversible solid/liquid change of state, having an anisotropic crystalline organization in the solid state, and including a liquid fraction and a solid fraction at a temperature of 23°C.

In other words, the initial melting point of the pasty compound may be less than 23°C. The liquid fraction of the pasty compound measured at 23°C may represent 9% to 97% by weight of the compound. This liquid fraction at 23°C preferably represents between 15% and 85%, more preferably between 40% and 85% by weight. According to the invention, the melting point is equivalent to the temperature of the most endothermic peak observed in thermal analysis (DSC) as described in the standard ISO 11357-3; 1999. The melting point of a paste or a wax may be measured using a differential scanning calorimeter (DSC), for example the calorimeter sold under the name "MDSC 2920" by 45 TA Instruments.

The measurement protocol is as follows: a 5 mg sample of paste or wax (according to the case) placed in a crucible is subjected to a first temperature rise from -20°C to 100°C, at a heating rate of 10°C / minute, and is then cooled from 100°C to -20°C at a cooling rate of 10°C / minute and finally subjected to a second temperature rise from -20°C to 100°C at a heating rate of 5°C / minute. During the second temperature rise, the variation in the difference in power absorbed by the empty crucible and by the crucible containing the paste or wax sample as a function of temperature is measured.

The melting point of the compound is the value of the temperature equivalent to the top point of the peak of the curve representing the variation in the difference in power absorbed as a function of temperature.

The liquid fraction by weight of the pasty compound at 23°C is equal to the ratio of the enthalpy of fusion consumed at 23°C to the enthalpy of fusion of the pasty compound. The enthalpy of fusion of the pasty compound is the enthalpy consumed by the compound to change from the solid state to the liquid state. The pasty compound is said to be in the solid state when the entire mass thereof is in solid crystalline form. The pasty compound is said to be in the liquid state when the entire mass thereof is in liquid form.

The enthalpy of fusion of the pasty compound is equal to the area under the curve of the thermogram obtained using differential scanning calorimeter (DSC), such as the calorimeter sold under the name MDSC 2920 by TA instrument, with a temperature rise of 5°C or 10°C per minute, according to the ISO 11357-3:1999 standard. The enthalpy of fusion of the pasty compound is the quantity of energy required to change the compound from the solid state to the liquid state. It is expressed in J/g. The enthalpy of fusion consumed at 23°C is the quantity of energy required by the sample to change from the solid state to the state presented at 23°C consisting of a liquid fraction and a solid fraction.

The liquid fraction of the pasty compound measured at 32°C preferably represents 30% to 100% by weight of the compound, preferably 50% to 100%, more preferably 60% to 100% by weight of the compound. If the liquid fraction of the pasty compound measured at 32°C is equal to 100%, the temperature of the end of the melting range of the pasty compound is less than or equal to 32°C.

The liquid fraction of the pasty compound at 32°C is equal to the ratio of the enthalpy of fusion consumed at 32°C to the enthalpy of fusion of the pasty compound. The enthalpy of fusion consumed at 32°C is calculated as for the enthalpy of fusion consumed at 23°C.

The pasty compound is preferably chosen from synthetic compounds and plant-based compounds. A pasty compound may be obtained by means of synthesis from plant-based starting materials. The pasty compound is advantageously chosen from:
- lanolin and the derivatives thereof;
- polyol ethers chosen from pentaerythritol and polyalkylene glycol ethers, fatty alcohol and sugar ethers, and mixtures thereof, pentaerythritol and polyethylene glycol ether comprising 5 oxyethylene units (5 OE) (CTFA name: PEG-5 Pentaerythrityl Ether), pentaerythritol and polypropylene glycol ether comprising 5 oxypropylene units (5 OP) (CTFA name: PPG-5 Pentaerythrityl Ether), and the mixtures thereof and more specifically the mixture of PEG-5 Pentaerythrityl Ether, PPG-5 Pentaerythrityl Ether and soybean oil, sold under the name "Lanolide" by Vevy, wherein the ratio of the constituents by weight is 46:46:8: 46% PEG-5 Pentaerythrityl Ether, 46% PPG-5 Pentaerythrityl Ether and 8% soybean oil;
- optionally polymeric silicone compounds;
- optionally polymeric fluorinated compounds;
- vinyl polymers, in particular olefin homopolymers and copolymers, hydrogenated diene homopolymers and copolymers, linear or branched oligomers, alkyl (meth)acrylate homo or copolymers preferably having a C8-C30 alkyl group, vinyl ester homo and copolymer oligomers, having C8-C30 alkyl groups, vinyl ether homo and copolymer oligomers, having C8-C30 alkyl groups;
- liposoluble polyethers derived from polyetherification between one or a plurality of C2-C100, preferably C2-C50, diols;
- esters;
- and/or mixtures thereof.

The pasty compound is preferably a polymer, in particular a hydrocarbon.

Of the liposoluble polyethers, preference is given in particular to ethylene-oxide and/or propylene-oxide copolymers with C6-C30 long-chain alkylene-oxides, more preferably such that the weight ratio of ethylene-oxide and/or propylene-oxide with alkylene-oxides in the copolymer is 5:95 to 70:30.

In this family, particular mention can be made of copolymers such as long-chain alkylene-oxides arranged in blocks having a mean molecular weight of 1000 to 10,000, for example a polyoxyethylene/polydodecyl glycol block copolymer such as the dodecanediol (22 mol) and polyethylene glycol (45 OE) ethers marketed under the brand ELFACOS ST9 by Akzo Nobel.

Among the esters, particular preference is given to:
- glycerol oligomer esters, namely diglycerol esters, particularly adipic acid and glycerol condensates, for which part of the hydroxyl groups of the glycerols have reacted with a mixture of fatty acids such as stearic acid, capric, stearic acid and isostearic acid and 12-hydroxystearic acid, particularly such as those sold under the brand Softisan 649 by Sasol;
- arachidyl propionate sold under the brand Waxenol 801 by Alzo;
- phytosterol esters;
- triglycerides of fatty acids and derivatives thereof;
- pentaerythritol esters;
- non-cross-linked polyesters derived from polycondensation between a dicarboxylic acid or a C4-C50 linear or branched carboxylic acid and a diol or an C2-C50 polyol;
- aliphatic esters of esters derived from the esterification of an aliphatic hydroxycarboxylic acid ester with an aliphatic carboxylic acid. Preferably, the aliphatic carboxylic acid comprises 4 to 30 and preferably 8 to 30 carbon atoms. It is preferably chosen from hexanoic acid, heptanoic acid, octanoic acid, ethyl-2 hexanoic acid, nonanoic acid, decanoic acid, undecanoic acid, dodecanoic acid, tridecanoic acid, tetradecanoic acid, pentadecanoic acid, hexadecanoic acid, hexyldecanoic acid, heptadecanoic acid, octadecanoic acid, isostearic acid, nonadecanoic acid, eicosanoic acid, isoarachidic acid, octyldodecanoic acid, heneicosanoic acid, docosanoic acid, and mixtures thereof. The aliphatic carboxylic acid is preferably branched. The aliphatic hydroxy carboxylic acid ester is advantageously derived from a hydroxylated aliphatic carboxylic acid comprising 2 to 40 carbon atoms, preferably 10 to 34 carbon atoms and more preferably 12 to 28 carbon atoms, and 1 to 20 hydroxyl groups, preferably 1 to 10 hydroxyl groups and more preferably 1 to 6 hydroxyl groups.

The aliphatic hydroxy carboxylic acid ester is in particular chosen from:
a) partial or total saturated, linear mono-hydroxylated aliphatic monocarboxylic acid esters;
b) partial or total unsaturated, linear mono-hydroxylated aliphatic monocarboxylic acid esters;
c) partial or total saturated non-hydroxylated aliphatic carboxylic polyacid esters;
d) partial or total saturated poly-hydroxylated aliphatic carboxylic polyacid esters;
e) partial or total C2 to C16 aliphatic polyol esters, having reacted with a mono or poly-hydroxylated aliphatic carboxylic mono or polyacid, and mixtures thereof.
   - dimer diol and dimer diacid esters, optionally esterified on the alcohol or free acid function(s) thereof by acid or alcohol radicals, particularly dimer dilinoleate esters, such esters may particularly be chosen from esters having the following INCI classification: bis-behenyl/isostearyl/phytosteryl dimerdilinoleyl dimerdilinoleate (Plandool G), phytosteryl/isosteryl/cetyl/stearyl/behenyl dimerdilinoleate (Plandool H or Plandool 40 S), and mixtures thereof;
   - hydrogenated rosinate esters, such as dimer dilinoleyl hydrogenated rosinates (Lusplan DD-DHR or DD-DHR from Nippon Fine Chemical); and
   - mixtures thereof.

### Waxes

According to one preferred embodiment, the composition A and/or B (or the ready-to-use composition or the single anhydrous composition) comprises at least one wax.

Generally, a wax considered in the framework of this invention is a lipophilic compound, which is solid at ambient temperature (25°C), having a reversible solid/liquid change of state and a melting point greater than or equal to 30°C of up to 200°C and particularly up to 120°C. In particular, the waxes suitable for the invention may have a melting point greater than or equal to 45°C, and particularly greater than or equal to 55°C. The waxes suitable for use in the compositions A and/or B (or the ready-to-use composition) are chosen from animal, plant, mineral or synthetic waxes, which are solid at ambient temperature, and mixtures thereof.

Mention can be made for example the following hydrocarbon waxes comprising a fatty alkyl chain having in general from 10 to 60 carbon atoms, preferably from 20 to 40 carbon atoms, said chain able to be saturated or unsaturated, substituted or not, linear, branched or cyclic, preferably saturated and linear:
- fatty alcohols;
- esters of fatty alcohols;
- fatty acids;
- fatty acid amides;
- fatty acid esters including triglycerides;
- fatty acid ethers;
- ethoxylated fatty alcohols;
- ethoxylated fatty alcohols, and the corresponding salts thereof.

Among the fatty alcohols, mention can be made of stearyl, cetearyl alcohol or mixtures thereof.

Among the esters of fatty alcohols, mention can be made of tri-isostearyl citrate, ethyleneglycol-di-12-hydroxystearate, tristearylcitrate, stearyl octanoate, stearyl heptanoate, trilauryl citrate and mixtures thereof. Among the fatty acid esters, mention can be made of ester waxes, monoglycerides, diglycerides, or triglycerides.

As an ester wax, mention can be made of stearyl stearate, stearyl behenate, stearyl octyldodecanol, cetearyl behenate, behenyl behenate, ethyleneglycol, distearate, ethyleneglycol dimaplimitate. It is possible to use in particular a C20-C40 alkyl (hydroxystearyloxy)stearate (the alkyl group comprising 20 to 40 carbon atoms), alone or in a mixture, may be used as the wax.

Such a wax is particularly sold under the names "Kester Wax K 82 P^{®}", "Hydroxypolyester K 82 P^{®}" and "Kester Wax K 80 P^{®}" by Koster Keunen.

Among the triglyceride waxes, mention can be made more particularly of tribehenin, C18-C36 triglyceride, and mixtures thereof.

By way of illustration of waxes suitable for the invention, particular mention can be made of hydrocarbon waxes such as beeswax, lanolin wax, and Chinese insect waxes, rice bran wax, carnauba wax, candelilla wax, ouricury wax, alfa wax, berry wax, shellac wax, Japan wax and sumac wax; montan wax, orange and lemon waxes, microcrystalline waxes, paraffins and ozokerite; polyethylene waxes, waxes obtained by means of Fisher-Tropsch synthesis and waxy copolymers and the esters thereof.

Mention can also be made of waxes obtained by means of the catalytic hydrogenation of animal or plant oils having C8-C32 linear or branched fat chains. Of these, particular mention can be made of isomerized jojoba oil such as the trans isomerized partially hydrogenated jojoba oil manufactured or sold by Desert Whale under the trade name Iso-Jojoba-50^{®}, hydrogenated sunflower oil, hydrogenated castor oil, hydrogenated coconut oil, hydrogenated lanolin oil, and di-(trimethylol-1,1,1 propane) tetrastearate sold under the name Hest 2T-4S^{®} by Heterene.

Mention can also be made of silicone waxes (C30-45 Alkyl dimethicone) and fluorinated waxes. It is also possible to use waxes obtained by hydrogenating esterified castor oil with cetyl alcohol sold under the names Phytowax ricin 16L64^{®} and 22L73^{®} by Sophim. Such waxes are in particular described in the application FR 2 792 190.

As micro-waxes that can be used in the compositions A and/or B, mention can be made in particular of the carnauba micro-waxes such as that marketed under the name MicroCare 350^{®} by Micro Powders, synthetic wax micro-waxes such as that marketed under the name MicroEase 114S^{®} by Micro Powders, micro-waxes formed from a mixture of carnauba wax and polyethylene wax such as those marketed under the names Micro Care 300^{®} and 310^{®} by Micro Powders, micro-waxes formed from a mixture of carnauba wax and of synthetic wax such as that marketed under the name Micro Care 325^{®} by Micro Powders, polyethylene micro-waxes such as those marketed under the names Micropoly 200^{®}, 220^{®}, 220L^{®} and 250S^{®} by Micro Powders and polytetrafluoroethylene micro-waxes such as those marketed under the names Microslip 519^{®} and 519 L^{®} by Micro Powders.

Composition A and/or B (or the ready-to-use composition or the single anhydrous composition) can comprise a solid fat content ranging preferably from 1% to 30% by weight, and in particular from 2% to 20% by weight relative to the total weight of the composition.

### Additives

Compositions A and/or B (or the ready-to-use composition or the single anhydrous composition) can further comprise additional cosmetic agents.

Cosmetic compositions A and/or B (or the ready-to-use composition or the single anhydrous composition) can furthermore comprise cosmetic additives chosen from among opacifiers, stabilizers, preservatives, perfume, solar filters, cosmetic active agents, fillers, suspension agents, sequestrants, coloring materials or any other ingredient routinely used in cosmetics for this type of application.

Obviously, a person skilled in the art will take care to choose the optional additional compound(s) in such a way that the advantageous properties intrinsically associated with composition A or B or with the ready-to-use composition or with the single anhydrous composition, are not altered, or are not substantially altered, by the envisaged additive(s).

According to a particular embodiment of the invention, compositions A and/or B (or the ready-to-use composition or the single anhydrous composition) will contain in addition an organic powder.

In this application, the term "organic powder" denotes any insoluble solid in the medium at ambient temperature (25°C).

As organic powders that can be used, mention can be made for example of polyamide particles, particularly those sold under the trade name ORGASOL by Atochem; nylon 6,6 fibers in particular the polyamide fibers marketed by Etablissements P Bonte under the name Polyamide 0.9 Dtex 0.3 mm (INCI name: Nylon 6,6 or Polyamide-6,6) having an average diameter of 6 µm, a weight of about 0.9 dtex and a length ranging from 0.3 mm to 1.5 mm; polyethylene powders; microspheres based on acrylic copolymers, such as those made of ethylene glycol dimethacrylate/lauryl methacrylate copolymer sold by Dow Corning under the trade name POLYTRAP; polymethyl methacrylate microspheres, marketed under the trade name MICROSPHERE M-100 by Matsumoto or under the trade name COVABEAD LH85 by Wackherr; hollow polymethyl methacrylate microspheres (granulometry: 6.5 - 10.5 µ) marketed under the trade name GANZPEARL GMP 0800 by Ganz Chemical; microbeads made of ethylene glycol dimethacrylate/lauryl methacrylate copolymer (size: 6.5-10.5 µ) marketed under the trade name GANZPEARL GMP 0820 by Ganz Chemical or MICROSPONGE 5640 by Amcol Health & Beauty Solutions; ethylene-acrylate copolymer powders, such as those marketed under the trade name FLOBEADS by Sumitomo Seika Chemicals; expanded powders such as hollow microspheres and in particular, the microspheres formed from a terpolymer of polyvinylidene/acrylonitrile/methacrylate and marketed under the trade name EXPANCEL by Kemanord Plast under the references 551 DE 12 (granulometry of about 12 µm and density 40 kg/m³), 551 DE 20 (granulometry of about 30 µm and density 65 kg/m³), 551 DE 50 (granulometry of about 40 µm), or the microspheres marketed under the trade name MICROPEARL F 80 ED by Matsumoto; powders of natural organic materials such as starch powders, in particular starch powders, in particular corn, wheat or rice starch, optionally cross-linked, such as starch powders cross-linked by octenylsuccinate anhydride, marketed under the trade name DRY-FLO by National Starch; silicone resin microbeads such as those marketed under the trade name TOSPEARL by Toshiba Silicone, in particular TOSPEARL 240; amino acid powders such as the lauroyllysine powder marketed under the trade name AMIHOPE LL-11 by Ajinomoto; particles of the microdispersion of wax, which preferably have average dimensions less than 1 µm and in particular ranging from 0.02 µm to 1 µm, and which are substantially comprised of a wax or of a mixture of waxes, such as the products marketed under the trade name Aquacer by Byk Cera, and in particular: Aquacer 520 (mixture of natural and synthetic waxes), Aquacer 514 or 513 (polyethylene wax), Aquacer 511 (polymer wax), or such as the products marketed under the trade name Jonwax 120 by Johnson Polymer (mixture of polyethylene waxes and of paraffin) and under the trade name Ceraflour 961 by Byk Cera (micronized modified polyethylene wax); and mixtures thereof.

According to an advantageous embodiment, composition A and/or B (or the ready-to-use composition) can further comprise at least one thickener. Such a thickener is preferably soluble or dispersible in water

The thickeners can be of natural or synthetic, mineral or organic origin.

The thickeners will preferably be anionic, zwitterionic or non-ionic polymers, associative or not.

The thickeners can be chosen from cellulose derivatives such as hydroxyethylcellulose; polysaccharides and in particular gums such as xanthan gum, sclerotium gum.

Associative polyurethanes are non-ionic sequenced copolymers comprising in the chain, both hydrophilic sequences generally polyoxyethylenated in nature (the polyurethanes can then be called polyether polyurethanes) and hydrophobic sequences which may be aliphatic chains alone and/or cycloaliphatic and/or aromatic chains.

In particular, these polymers comprise at least two hydrocarbon lipophilic chains, having 6 to 30 carbon atoms, separated by a hydrophilic sequence, the hydrocarbon chains may be pendant chains or hydrophilic sequence end chains. In particular, one or a plurality of pendant chains may be envisaged. Moreover, the polymer may comprise a hydrocarbon chain at one end or at both ends of a hydrophilic sequence.

Associative polyurethanes may be sequenced in triblock or multiblock form. The hydrophobic sequences may thus be at each end of the chain (for example: triblock copolymer having hydrophilic central sequence) or distributed both at the end and in the chain (multisequenced copolymer for example). These polymers may also be grafted or star polymers. Preferably, associative polyurethanes are triblock copolymers of which the hydrophilic sequence is a polyoxyethylenated chain comprising 50 to 1000 oxyethylenated groups. In general, associative polyurethanes comprise a urethane bond between the hydrophilic sequences, hence the name.

According to an embodiment, use is made as a gelling agent of a non-ionic associative polymer of the polyurethane type.

As examples of fatty-chain non-ionic polyether polyurethanes that can be used in the invention, use can also be made of Rheolate FX 1100 or Rheoluxe 811 (Steareth-100/PEG-136/HDI(hexamethyl diisocyanate) copolymer), Rheolate 205 with urea function sold by ELEMENTIS or Rheolates 208, 204 or 212, as well as Acrysol RM 184 or Acrysol RM 2020.

Mention can also be made of the product ELFACOS T210 with C12-C14 alkyl chain and the product ELFACOS T212 with C16-18 alkyl chain (PPG-14 Palmeth-60 Hexyl Dicarbamate) from AKZO.

The product DW 1206B from ROHM & HAAS with C20 alkyl chain and urethane bond, proposed at 20% dry material in water, can also be used.

Solutions or dispersions of these polymers can also be used in particular in water or in a hydroalcoholic medium. As an example of such polymers, mention can be made of RHEOLATE 255, RHEOLATE 278 and RHEOLATE 244 sold by ELEMENTIS. The DW 1206F product and the DW 1206J product proposed by ROHM & HAAS can also be used.

The associative polyurethanes that can be used according to the invention are in particular those described in the article by G. Fonnum, J. Bakke and Fk. Hansen - Colloid Polym. Sci 271, 380.389 (1993).

Use may also be made of an associative polyurethane suitable for being obtained by means of polycondensation of at least three compounds comprising (i) at least one polyethylene glycol comprising 150 to 180 moles of ethylene oxide, (ii) stearyl alcohol or decyl alcohol and (iii) at least one diisocyanate.

Such polyether polyurethanes are sold in particular by ROHM & HAAS under the names ACULYN 46 and ACULYN 44 [ACULYN 46 is a polycondensate of polyethyleneglycol at 150 or 180 moles of ethylene oxide, stearyl alcohol and methylene bis(4-cyclohexylisocyanate) (SMDI), at 15% by weight in a matrix of maltodextrin (4%) and water (81%); ACULYN 44 is a polycondensate of polyethyleneglycol at 150 or 180 moles of ethylene oxide, of decyl alcohol and of methylene bis(4-cyclohexylisocyanate) (SMDI), at 35% by weight in a mixture of propyleneglycol (39%) and of water (26%)].

Solutions or dispersions of these polymers can also be used in particular in water or in a hydroalcoholic medium. As an example of such polymers, mention can be made of RHEOLATE FX1010, RHEOLATE FX1035 and RHEOLATE 1070 from ELEMENTIS, RHEOLATE 255, RHEOLATE 278 and RHEOLATE 244 sold by ELEMENTIS. It is also possible to use the products ACULYN 44, ACULYN 46, DW 1206F and DW 1206J, as well as Acrysol RM 184 from ROHM & HAAS, or BORCHI GEL LW 44 from BORCHERS, and mixtures thereof.

Preferably, a non-ionic associative polyether polyurethane is used such as the one sold in particular by ELEMENTIS under the name RHEOLATE FX 1100 or RHEOLUXE 811 which is a polycondensate of polyethyleneglycol at 136 moles of ethylene oxide, of polyoxyethylenated stearyl alcohol at 100 moles of ethylene oxide and of hexamethylene diisocyanate (HDI) having a mean molecular weight by weight of 30,000 (INCI name: Steareth-100/ PEG-136/HDI Copolymer).

The thickeners are generally present in composition A and/or B (or in the ready-to-use composition) at a content ranging from 0% to 20% by weight, preferably from 0% to 10% by weight, and most preferably from 0% to 7% by weight, relative to the total weight of the composition.

The composition can also comprise at least one lipophilic gelling agent. By way of example of a mineral lipophilic gelling agent, mention can be made of optionally modified clays such as hectorites modified by a C10 to C22 ammonium chloride, such as hectorite modified with di-stearyl di-methyl ammonium chloride such as, for example, that marketed under the name Bentone 38V by ELEMENTIS.

Mention can also be made of pyrogenic silica optionally with a hydrophobic surface treatment wherein the particle size is less than 1 µm. Indeed, it is possible to modify the surface of the silica chemically, by means of a chemical reaction giving rise to a reduction in the silanol groups present on the silica surface. The silanol groups can particularly be substituted with hydrophobic groups: a hydrophobic silica is thus obtained. The hydrophobic groups can be trimethylsiloxyl groups, particularly obtained by treating pyrogenic silica in the presence of hexamethyldisilazane. Silicas treated in this way are referred to as "Silica silylate" as per the CTFA (8th edition, 2000). They are for example marketed under the references Aerosil R812 by DEGUSSA, CAB-O-SIL TS-530 by CABOT, dimethylsilyloxyl or polydimethylsiloxane groups, particularly obtained by treating pyrogenic silica in the presence of polydimethylsiloxane or dimethyldichlorosilane. Silicas treated in this way are referred to as "Silica dimethyl silylate" as per the CTFA (8th edition, 2000). They are for example sold under the references Aerosil R972, and Aerosil R974 by DEGUSSA, CAB-O-SIL TS-610 and CAB-O-SIL TS-720 by CABOT.

The hydrophobic pyrogenic silica particularly has a particle size that may be nanometric to micrometric, for example ranging from approximately 5 to 200 nm.

The polymeric organic lipophilic gelling agents are, for example, partially or totally cross-linked elastomeric organopolysiloxanes with a three-dimensional structure, such as those sold under the names KSG6, KSG16 and KSG18 by SHIN-ETSU, Trefil E-505C and Trefil E-506C by DOW-CORNING, Gransil SR-CYC, SR DMF10, SR-DC556, SR 5CYC gel, SR DMF 10 gel and SR DC 556 gel by GRANT INDUSTRIES, SF 1204 and JK 113 by GENERAL ELECTRIC; ethylcellulose such as that sold under the trade name Ethocel by DOW CHEMICAL; galactomannans containing from one to six, and in particular from two to four, hydroxyl groups per monosaccharide, substituted with a saturated or unsaturated alkyl chain, such as guar gum alkylated with C1 to C6 alkyl chains, and C1 to C3 in particular and mixtures thereof. Block copolymers of the "diblock", "triblock" or "radial" type, of the polystyrene/polyisoprene or polystyrene/polybutadiene type, such as those marketed under the trade name Luvitol HSB^{®} by BASF, of the polystyrene/copoly(ethylene-propylene) type, such as those marketed under the name Kraton^{®} by SHELL CHEMICAL CO or of the polystyrene/copoly(ethylene-butylene) type, mixtures of triblock and radial (star) copolymers in isododecane, such as those marketed by PENRECO under the trade name Versagel^{®} for instance the mixture of butylene/ethylene/styrene triblock copolymer and of ethylene/propylene/styrene star copolymer in isododecane (Versagel M 5960).

As a lipophilic gelling agent, mention can further be made of polymers of mean molar mass by weight less than 100,000, comprising a) a polymeric backbone that has hydrocarbon repeat units provided with at least one heteroatom, and optionally b) at least one pendant fatty chain and/or at least one terminal fatty chain that may be functionalized, having from 6 to 120 carbon atoms and being linked to these hydrocarbon patterns, such as those described in applications WO-A-02/056847, WO-A-02/47619 in particular resins of polyamides (in particular comprising alkyl groups having from 12 to 22 carbon atoms) such as those described in US-A-5783657.

Among the lipophilic gelling agents suitable for use, mention may also be made of dextrin and fatty acid esters, such as dextrin palmitates, particularly such as those sold under the names Rheopearl TL or Rheopearl KL by CHIBA FLOUR.

Use can also be made of polyorganosiloxane type silicone polyamides such as those described in the documents US-A-5,874,069, US-A-5,919,441, US-A-6,051,216 and US-A-5,981,680.

These silicone polyamides can belong to the following two families:
- polyorganosiloxanes including at least two amide groups capable of establishing hydrogen interactions, with these two groups being located in the chain of the polymer, and/or
- polyorganosiloxanes including at least two amide groups capable of establishing hydrogen interactions, with these two groups being located on grafts or branches.

Compositions A and/or B (or the ready-to-use composition or the single anhydrous composition) can furthermore contain one or more suspension agents and/or one or more gelling agents. Some of them can play both roles at the same time.

Among the agents that can be used as a suspension agent and/or as a lipophilic gelling agent, mention can be made of clays, in the form of powder or in the form of an oily gel; said clays optionally able to be modified in particular modified montmorillonite clays such as modified hydrophobic bentonites or hectorites such as the hectorites modified by a C10 to C22 ammonium chloride, such as hectorite modified with di-stearyl di-methyl ammonium chloride such as, for example, the product Disteardimonium Hectorite (CTFA name) (reaction product of hectorite and of disteardimonium chloride) sold under the name of Bentone 38 or Bentone Gel by Elementis Specialities. Mention can be made for example of the product Stearalkonium Bentonite (CTFA name) (reaction product of bentonite and stearalkonium chloride quaternary ammonium) such as the commercial product sold under the name TIXOGEL MP 250^{®} by Sud Chemie Rheologicals, United Catalysts Inc.

Use can also be made of hydrotalcites, in particular modified hydrophobic hydrotalcites such as for example the products sold under the name of Gilugel by BK Giulini.

Mention can also be made of pyrogenic silica optionally with a hydrophobic surface treatment wherein the particle size is less than 1 µm. Indeed, it is possible to modify the surface of the silica chemically, by means of a chemical reaction giving rise to a reduction in the silanol groups present on the silica surface. The silanol groups can particularly be substituted with hydrophobic groups: a hydrophobic silica is thus obtained. The hydrophobic groups can be trimethylsiloxyl groups, particularly obtained by treating pyrogenic silica in the presence of hexamethyldisilazane. Silicas treated in this way are referred to as "Silica silylate" as per the CTFA (8th edition, 2000). They are for example marketed under the references Aerosil R812 by DEGUSSA, CAB-O-SIL TS-530^{®} by CABOT, dimethylsilyloxyl or polydimethylsiloxane groups, particularly obtained by treating pyrogenic silica in the presence of polydimethylsiloxane or dimethyldichlorosilane. Silicas treated in this way are referred to as "Silica dimethyl silylate" as per the CTFA (8th edition, 2000). They are for example sold under the references Aerosil R972, and Aerosil R974 by DEGUSSA, CAB-O-SIL TS-610 and CAB-O-SIL TS-720 by CABOT.

The hydrophobic pyrogenic silica particularly has a particle size that may be nanometric to micrometric, for example ranging from approximately 5 to 200 nm.

According to a particular form of the invention, the suspension agents or gelling agents can be activated by oils such as propylene carbonate, triethylcitrate.

The quantities of these different constituents that can be present in composition A and/or B (or in the ready-to-use composition or in the single anhydrous composition) are those conventionally used in compositions for the treatment of perspiration.

The suspension agents are present preferably in quantities ranging from 0.1% to 5% by weight and more preferably from 0.2 to 2% by weight relative to the total weight of the composition.

The quantities of these different constituents that can be present in cosmetic compositions A and/or B (or in the ready-to-use composition or the single anhydrous composition) are those conventionally used in compositions for the treatment of perspiration.

### AEROSOLS

Compositions A and/or B can also be pressurized and be packaged in an aerosol device, in particular comprising at least one propellant.

The propellant used is chosen preferably from dimethylether, volatile hydrocarbons such as propane, isopropane, n-butane, isobutane, n-pentane and isopentane and mixtures thereof, optionally with at least one chlorinated and/or fluorinated hydrocarbon; of the latter, mention can be made of the compounds sold by Dupont de Nemours under the trade names Freon and Dymel, and in particular monofluorotrichloromethane, difluorodichloromethane, tetrafluorodichloroethane and 1,1-difluoroethane particularly sold under the trade name DYMEL 152 A^{®} by DUPONT.

Carbon dioxide, nitrous oxide, nitrogen or compressed air may also be used as a propellant. Preferably, the propellant is chosen from the volatile hydrocarbons.

More preferably, the propellant is chosen from isopropane, n-butane, isobutane, pentane and isopentane and mixtures thereof.

The weight ratio between the liquid phase and the propellant gas varies in a ratio from 5/95 to 50/50, preferably from 10/90 to 40/60, and more preferably from 15/85 to 30/70.

According to the invention, the propellant concentration generally varies from 5 to 95% by pressurized weight and more preferentially from 50 to 85% by weight relative to the total weight of the pressurized A and/or B composition.

The dispensing means, forming part of the aerosol device, generally consist of a dispensing valve controlled by a dispensing head, in turn comprising a nozzle via which the mixture of composition A and of composition B is sprayed. The receptacle containing each pressurized composition A and B may be opaque or transparent. It can be made of glass, polymeric or metal material, optionally coated with a layer of protective varnish.

The expressions "between ... and ..." and "ranging from ... to ..." are to be understood to be inclusive of the limits, unless specified otherwise.

In the description and the examples, unless mentioned otherwise, the percentages are weight percentages. The temperature is expressed in degrees Celsius unless mentioned otherwise, and the pressure is the atmospheric pressure, unless mentioned otherwise. The invention is illustrated in more detail by the non-limiting examples presented hereinafter.

The examples that follow are used to illustrate this invention. The quantities are indicated as a percentage by weight relative to the total weight of the composition (% w/w).

### Example 1: Anhydrous dosage forms according to the invention

### Formula 1A: aerosol composition:

The following composition 1A according to the invention is obtained by mixing MgO and KH₂PO₄ dispersed in oils:

**[Table 1]**

| **Ingredient** | **Quantity (% w/w)** |
|---|---|
| Coconut oil (Cocos Nucifera oil) | 26.8 |
| Isopropyl palmitate | 60 |
| KH₂PO₄ (molar mass = 136 g/mol) | 10 (i.e. 0.0735 moles) |
| MgO (molar mass = 40.3 g/mol) | 3 (i.e. 0.0744 moles) |

The KH₂PO₄/MgO molar ratio is 0.988.

### Formula 1B: Power dispersion:

Composition 1B according to the invention can be obtained by mixing dry powders (optionally compacted) of MgO (for example 3% by weight) and KH₂PO₄ (for example 10% by weight), with starch (filler, qs 100), with or without binder such as magnesium stearate (for example 1% to 20% by weight).

### Formula 1C: anhydrous stick:

Composition 1C according to the invention can be obtained by mixing MgO (for example 3% by weight) and KH₂PO₄ (for example 10% by weight) dispersed in a polyethylene wax (for example 10% by weight) in an oil (qs 100).

### Formula 1D: anhydrous glycol stick

Composition 1D according to the invention is obtained by mixing MgO and KH₂PO₄ dispersed in the mixture described in table 2:

**[Table 2]**

| **Ingredient** | **Quantity (% w/w)** |
|---|---|
| Glycerol | 20 |
| Propylene Glycol | 58 |
| Steareth 100 | 2 |
| Magnesium stearate | 8 |
| KH₂PO₄ (molar mass = 136 g/mol) | 15.4 (i.e. 0.113 moles) |
| MgO (molar mass = 40.3 g/mol) | 4.6 (i.e. 0.114 moles) |

The KH₂PO₄/MgO molar ratio is 0.99.

### Formulas 1: Anhydrous glycolic or hydroglycolic gels:

The compositions according to the invention can be obtained:
- by mixing MgO (for example 3% by weight) incorporated in a mixture of propylene glycol and glycerin with or without water (between 10 and 15%);
- by mixing KH₂PO₄ (for example 10% by weight) incorporated in a mixture of propylene glycol and glycerin with or without water (between 10 and 15%).

### Example 2: Aqueous dosage forms according to the invention

### Formulas 2A: Aqueous gels:

The compositions according to the invention can be obtained:
- by mixing MgO (for example 3% by weight) incorporated in an aqueous gel (water qs 100) comprising a gelling agent such as hydroxyethylcellulose or xanthan gum (in a quantity between 1 and 5% by weight), to obtain a first aqueous gel; and
- by mixing KH₂PO₄ (for example 10% by weight) incorporated in an aqueous gel (water qs 100) comprising a gelling agent such as hydroxyethylcellulose or xanthan gum (in a quantity between 1 and 5% by weight), to obtain a second aqueous gel.

### Formulas 2B: Hydroalcoholic gels:

The compositions according to the invention can be obtained:
- by mixing MgO (for example 3% by weight) incorporated in a hydroalcoholic gel (ethanol in a quantity between 20% and 30% by weight, and water qs 100) comprising a gelling agent such as hydroxyethylcellulose or xanthan gum (in a quantity ranging from 1% to 5% by weight), to obtain a first hydroalcoholic gel; and
- by mixing KH₂PO₄ (for example 10% by weight) incorporated in a hydroalcoholic gel (ethanol in a quantity between 20% and 30% by weight, and water qs 100) comprising a gelling agent such as hydroxyethylcellulose or xanthan gum (in a quantity ranging from 1% to 5% by weight), to obtain a second hydroalcoholic gel.

### Formulas 2C: Fluid emulsions:

The compositions according to the invention can be obtained:
- by mixing MgO (for example 3% by weight) incorporated in a direct or invert emulsion comprising an oily phase comprising 2 to 10% by weight of silicone oil such as dimethicone and/or hydrocarbon oil such as isopropylpalmitate, and at least one emulsifier such as steareth, in order to obtain a first emulsion; and
- by mixing KH₂PO₄ (for example 10% by weight) incorporated in a direct or invert emulsion comprising an oily phase comprising 2 to 10% by weight of silicone oil such as dimethicone and/or hydrocarbon oil such as isopropylpalmitate, and at least one emulsifier such as steareth, in order to obtain a second emulsion.

### Formula 2D: hydroglycolic stick

Composition 2D according to the invention is obtained by mixing MgO and KH₂PO₄ dispersed in the two separate mixtures described in the tables below:

**[Table 3]**

| **Ingredient** | **Quantity (% w/w)** |
|---|---|
| Propylene Glycol | 55 |
| Glycerin | 20 |
| Sodium stearate | 8 |
| Oxyethylenated stearyl alcohol (100 OE) | 2 |
| MgO | 3 |
| Water | Qs 100 |

**[Table 4]**

| **Ingredient** | **Quantity (% w/w)** |
|---|---|
| Propylene Glycol | 48 |
| Glycerin | 20 |
| Sodium stearate | 8 |
| Oxyethylenated stearyl alcohol (100 OE) | 2 |
| KH₂PO₄ | 10 |
| Water | Qs 100 |

### Example 3: Influence of KH₂PO₄/MgO molar ratio on deodorant effectiveness

The deodorant effectiveness of different KH₂PO₄/MgO mixtures at different molar ratios was studied *in vitro* in a "sniff test" model using human sweat incubated at 37°C.

Table 2 below shows the average intensity rating results of the odor (scores from 1 = weak odor to 8 = very strong odor) perceived *in vitro* according to the KH₂PO₄/MgO molar ratio:

**[Table 5]**

| **Test product** | **KH₂PO₄/MgO molar ratio** | **Odor intensity** |
|---|---|---|
| Control | - | 8 |
| MgO | - | 1 |
| KH₂PO₄ | - | 8 |
| MgO + KH₂PO₄ (invention) | 0.33 | 1 |
| MgO + KH₂PO₄ (invention) | 0.67 | 2 |
| MgO + KH₂PO₄ (comparative) | 1 | 7 |

It is clear that the deodorant activity of the mixtures is high for molar ratios of 0.33 and 0.67, but lost for a molar ratio of 1 (complete consumption of MgO).

## Claims

1. Cosmetic process for treating human perspiration and treating body odors resulting from perspiration, comprising the use of magnesium oxide and at least one water-soluble phosphate acid salt that is potassium dihydrogen phosphate, the molar ratio between the water-soluble phosphate acid salt and magnesium oxide being strictly less than 1.

2. Process according to claim 1, **characterized in that** the water-soluble phosphate acid salt and magnesium oxide are formulated in anhydrous medium within the same composition.

3. Process according to claim 2, **characterized in that** the composition further comprises at least one modulating agent chosen from:
- mono or polycarboxylic acids, optionally hydroxylated, in free or salified form,
- amino carboxylic acids in free or salified form,
- monosaccharides, oligosaccharides, polysaccharides and the derivatives thereof,
- ascorbic acid,
- phytic acid,
- polymers or copolymers of carboxylic acids in free or salified form,
- polymers or copolymers of amino carboxylic acids in free or salified form,
- polymers or copolymers of maleic or itaconic acid, and
- polymers or copolymers of carboxymethylinulin.

4. Process according to claim 1, **characterized in that**:
- magnesium oxide is present in a composition A; and
- the water-soluble phosphate acid salt is present in a composition B, compositions A and B being different.

5. Process according to claim 4, **characterized in that** at least one modulating agent is present in composition A and/or in composition B.

6. Process according to one of claims 1 to 5, comprising:
either the mixing just before use of at least one composition A and of at least one composition B, said compositions A and B being packaged separately, followed by the application of the resulting mixture on the surface of the skin;
or the application on the surface of the skin simultaneously or sequentially of at least one composition A and of at least one composition B packaged separately;
or the application on the surface of the skin of a composition comprising in the same holder at least one composition A and of at least one composition B;
or the application on the surface of the skin of a composition, preferably anhydrous, comprising magnesium oxide and a water-soluble phosphate acid salt, provided that said magnesium oxide and said salt are not in contact with one another in said composition.

7. Process according to one of claims 1 to 6, **characterized in that** magnesium oxide is present at a quantity ranging from 1 to 20% by weight and more preferably from 2 to 10% by weight relative to the total weight of the composition(s).

8. Process according to one of claims 1 to 7, **characterized in that** the water-soluble phosphate acid salt is present at a quantity ranging from 1 to 30% by weight and more preferably from 2 to 20% by weight relative to the total weight of the composition(s).

9. Process according to one of claims 3 or 5 to 8, **characterized in that** the modulating salt is chosen from:
- mono or polycarboxylic acids, optionally hydroxylated, in free or salified form, chosen from propionic acid, citric acid, tartaric acid, lactic acid, malic acid, succinic acid, glutaric acid, itaconic acid,
- amino carboxylic acids in free or salified form chosen from aspartic acid, glutamic acid, serine, alanine, dehydroalanine and the oligomers thereof, iminosuccinic acid and the derivatives thereof, ethylene diamine tetraacetic acid,
- monosaccharides, oligosaccharides, polysaccharides and the derivatives thereof, chosen from glucose, galactose, mannose, xylose, lyxose, fucose, arabinose, rhamnose, ribose, deoxyribose, quinovose, fructose, sorbose, talose, threose, erythrose, trehalose, lactose, maltose, cellobiose, glucuronic acid, lactobionic acid, alginates, chitosans and pectins,
- ascorbic acid,
- phytic acid,
- polymers or copolymers of carboxylic acids in free or salified form,
- polymers or copolymers of amino carboxylic acids in free or salified form chosen from polyaspartic acid and polyglutamic acid,
- polymers or copolymers of maleic or itaconic acid, and
- polymers or copolymers of carboxymethylinulin.

10. Process according to one of claims 1 to 9, **characterized in that** the composition(s) comprise less than 5% by weight of aluminum salt, preferably less than 3% by weight, preferably less than 1% by weight, and preferably are totally free from aluminum salt.

11. Process according to one of claims 1 to 10, **characterized in that** the molar ratio between the water-soluble phosphate acid salt and magnesium oxide is less than or equal to 0.9, preferably less than or equal to 0.8, preferably between 0.2 and 0.9, preferably between 0.3 and 0.8.

12. Ready-to-use cosmetic composition, particularly for treating human perspiration and treating body odors resulting from perspiration, which comprises, in a cosmetically acceptable medium, magnesium oxide and at least one water-soluble phosphate acid salt that is potassium dihydrogen phosphate, the molar ratio between the water-soluble phosphate acid salt and magnesium oxide being strictly less than 1.

13. Cosmetic composition according to claim 12, **characterized in that** the molar ratio between the water-soluble phosphate acid salt and magnesium oxide is less than or equal to 0.9, preferably less than or equal to 0.8, preferably between 0.2 and 0.9, preferably between 0.3 and 0.8.

## Patentansprüche

1. Kosmetisches Verfahren zum Behandeln von menschlicher Transpiration und zum Behandeln von Körpergerüchen, die aus der Transpiration resultieren, umfassend die Verwendung von Magnesiumoxid und mindestens einem wasserlöslichen Phosphatsäuresalz, das Kaliumdihydrogenphosphat ist, wobei das molare Verhältnis zwischen dem wasserlöslichen Phosphatsäuresalz und dem Magnesiumoxid strikt kleiner als 1 ist.

2. Verfahren nach Anspruch 1, **dadurch gekennzeichnet, dass** das wasserlösliche Phosphatsäuresalz und Magnesiumoxid in wasserfreiem Medium in derselben Zusammensetzung formuliert sind.

3. Verfahren nach Anspruch 2, **dadurch gekennzeichnet, dass** die Zusammensetzung ferner mindestens ein modulierendes Mittel umfasst, das ausgewählt ist aus:
- Mono- oder Polycarbonsäuren, optional hydroxyliert, in freier Form oder Salzform,
- Aminocarbonsäuren in freier Form oder Salzform,
- Monosacchariden, Oligosacchariden, Polysacchariden und deren Derivaten,
- Ascorbinsäure,
- Phytinsäure,
- Polymeren oder Copolymeren von Carbonsäuren in freier Form oder Salzform,
- Polymeren oder Copolymeren von Aminocarbonsäuren in freier Form oder Salzform,
- Polymeren oder Copolymeren von Maleinsäure oder Itaconsäure, und
- Polymeren oder Copolymeren von Carboxymethylinulin.

4. System nach Anspruch 1, **dadurch gekennzeichnet, dass**:
- Magnesiumoxid in einer Zusammensetzung A vorhanden ist; und
- das wasserlösliche Phosphatsäuresalz in einer Zusammensetzung B vorhanden ist, wobei die Zusammensetzungen A und B verschieden sind.

5. Verfahren nach Anspruch 4, **dadurch gekennzeichnet, dass** mindestens ein modulierendes Mittel in Zusammensetzung A und/oder in Zusammensetzung B vorhanden ist.

6. Verfahren nach einem der Ansprüche 1 bis 5, umfassend:
entweder das Mischen von mindestens einer Zusammensetzung A und mindestens einer Zusammensetzung B unmittelbar vor der Anwendung, wobei die Zusammensetzungen A und B getrennt verpackt sind, gefolgt von dem Auftragen des resultierenden Gemischs auf die Hautoberfläche;
oder das gleichzeitige oder aufeinanderfolgende Auftragen mindestens einer Zusammensetzung A und mindestens einer separat verpackten Zusammensetzung B auf die Hautoberfläche;
oder das Auftragen einer Zusammensetzung auf die Hautoberfläche, die in demselben Behälter mindestens eine Zusammensetzung A und mindestens eine Zusammensetzung B enthält;
oder das Auftragen einer vorzugsweise wasserfreien Zusammensetzung, umfassend Magnesiumoxid und ein wasserlösliches Phosphatsäuresalz, auf die Hautoberfläche, mit der Maßgabe, dass das Magnesiumoxid und das Salz in der Zusammensetzung nicht miteinander in Kontakt sind.

7. Verfahren nach einem der Ansprüche 1 bis 6, **dadurch gekennzeichnet, dass** Magnesiumoxid in einer Menge in einem Bereich von 1 bis 20 Gewichts-% und vorzugsweise von 2 bis 10 Gewichts-%, bezogen auf das Gesamtgewicht der Zusammensetzung(en), vorhanden ist.

8. Verfahren nach einem der Ansprüche 1 bis 7, **dadurch gekennzeichnet, dass** das wasserlösliche Phosphatsäuresalz in einer Menge in einem Bereich von 1 bis 30 Gewichts-% und vorzugsweise von 2 bis 20 Gewichts-%, bezogen auf das Gesamtgewicht der Zusammensetzung(en), vorhanden ist.

9. Verfahren nach einem der Ansprüche 3 oder 5 bis 8, **dadurch gekennzeichnet, dass** das modulierende Salz ausgewählt ist aus:
- Mono- oder Polycarbonsäuren, optional hydroxyliert, in freier Form oder in Salzform, ausgewählt aus Propionsäure, Zitronensäure, Weinsäure, Milchsäure, Äpfelsäure, Bernsteinsäure, Glutarsäure, Itaconsäure,
- Aminocarbonsäuren in freier Form oder in Salzform, ausgewählt aus Asparaginsäure, Glutaminsäure, Serin, Alanin, Dehydroalanin und deren Oligomeren, Iminobernsteinsäure und Derivaten davon, Ethylendiamintetraessigsäure,
- Monosaccharide, Oligosaccharide, Polysaccharide und Derivaten davon, ausgewählt aus Glucose, Galactose, Mannose, Xylose, Lyxose, Fucose, Arabinose, Rhamnose, Ribose, Desoxyribose, Quinovose, Fructose, Sorbose, Talose, Threose, Erythrose, Trehalose, Lactose, Maltose, Cellobiose, Glucuronsäure, Lactobionsäure, Alginate, Chitosane und Pektine,
- Ascorbinsäure,
- Phytinsäure,
- Polymeren oder Copolymeren von Carbonsäuren in freier Form oder Salzform,
- Polymere oder Copolymere von Aminocarbonsäuren in freier Form oder in Salzform, ausgewählt aus Polyasparaginsäure und Polyglutaminsäure,
- Polymeren oder Copolymeren von Maleinsäure oder Itaconsäure, und
- Polymeren oder Copolymeren von Carboxymethylinulin.

10. Verfahren nach einem der Ansprüche 1 bis 9, **dadurch gekennzeichnet, dass** die Zusammensetzung(en) weniger als 5 Gewichts-% Aluminiumsalz, vorzugsweise weniger als 3 Gewichts-%, vorzugsweise weniger als 1 Gewichts-%, umfassen und vorzugsweise völlig frei von Aluminiumsalz sind.

11. Verfahren nach einem der Ansprüche 1 bis 10, **dadurch gekennzeichnet, dass** das molare Verhältnis zwischen dem wasserlöslichen Phosphatsäuresalz und dem Magnesiumoxid kleiner als oder gleich wie 0,9, vorzugsweise kleiner als oder gleich wie 0,8, vorzugsweise zwischen 0,2 und 0,9, vorzugsweise zwischen 0,3 und 0,8 ist.

12. Gebrauchsfertige kosmetische Zusammensetzung, insbesondere zum Behandeln von menschlicher Transpiration und des aus Transpiration resultierenden Körpergeruchs, die in einem kosmetisch annehmbaren Medium Magnesiumoxid und mindestens ein wasserlösliches Phosphatsäuresalz, das Kaliumdihydrogenphosphat ist, umfasst, wobei das molare Verhältnis zwischen dem wasserlöslichen Phosphatsäuresalz und dem Magnesiumoxid streng unter 1 ist.

13. Kosmetische Zusammensetzung nach Anspruch 12, **dadurch gekennzeichnet, dass** das molare Verhältnis zwischen dem wasserlöslichen Phosphatsäuresalz und dem Magnesiumoxid 0,9, vorzugsweise kleiner oder gleich 0,8, vorzugsweise zwischen 0,2 und 0,9, vorzugsweise zwischen 0,3 und 0,8 ist.

## Revendications

1. Procédé cosmétique pour traiter la transpiration humaine et traiter les odeurs corporelles résultant de la transpiration, comprenant l'utilisation d'oxyde de magnésium et d'au moins un sel d'acide phosphate soluble dans l'eau qui est le dihydrogénophosphate de potassium, le rapport molaire entre le sel d'acide phosphate soluble dans l'eau et l'oxyde de magnésium étant strictement inférieur à 1.

2. Procédé selon la revendication 1, **caractérisé en ce que** le sel d'acide phosphate soluble dans l'eau et l'oxyde de magnésium sont formulés en milieu anhydre dans la même composition.

3. Procédé selon la revendication 2, **caractérisé en ce que** la composition comprend en outre au moins un agent modulateur choisi parmi :
- les acides mono ou polycarboxyliques, éventuellement hydroxylés, sous forme libre ou salifiée,
- les acides aminocarboxyliques sous forme libre ou salifiée,
- les monosaccharides, oligosaccharides, polysaccharides et leurs dérivés,
- l'acide ascorbique,
- l'acide phytique,
- les polymères ou copolymères d'acides carboxyliques sous forme libre ou salifiée,
- les polymères ou copolymères d'acides aminocarboxyliques sous forme libre ou salifiée,
- les polymères ou copolymères de l'acide maléique ou itaconique, et
- les polymères ou copolymères de carboxyméthylinuline.

4. Procédé selon la revendication 1, **caractérisé en ce que** :
- l'oxyde de magnésium est présent dans une composition A ; et
- le sel d'acide phosphate soluble dans l'eau est présent dans une composition B, les compositions A et B étant différentes.

5. Procédé selon la revendication 4, **caractérisé en ce qu'**au moins un agent modulateur est présent dans la composition A et/ou dans la composition B.

6. Procédé selon l'une quelconque des revendications 1 à 5, comprenant :
soit le mélange juste avant utilisation d'au moins une composition A et d'au moins une composition B, lesdites compositions A et B étant conditionnées séparément, suivi de l'application du mélange obtenu sur la surface de la peau ;
soit l'application sur la surface de la peau, simultanément ou séquentiellement, d'au moins une composition A et d'au moins une composition B conditionnées séparément ;
soit l'application sur la surface de la peau d'une composition comprenant dans le même support au moins une composition A et au moins une composition B ;
soit l'application sur la surface de la peau d'une composition, de préférence anhydre, comprenant de l'oxyde de magnésium et un sel d'acide phosphate soluble dans l'eau, à condition que ledit oxyde de magnésium et ledit sel ne soient pas en contact l'un avec l'autre dans ladite composition.

7. Procédé selon l'une quelconque des revendications 1 à 6, **caractérisé en ce que** l'oxyde de magnésium est présent en une quantité allant de 1 à 20 % en poids et plus préférentiellement de 2 à 10 % en poids par rapport au poids total de la (des) composition(s).

8. Procédé selon l'une quelconque des revendications 1 à 7, **caractérisé en ce que** le sel d'acide phosphate soluble dans l'eau est présent en une quantité allant de 1 à 30 % en poids et plus préférentiellement de 2 à 20 % en poids par rapport au poids total de la (des) composition(s).

9. Procédé selon l'une quelconque des revendications 3 ou 5 à 8, **caractérisé en ce que** le sel modulateur est choisi parmi :
- les acides mono ou polycarboxyliques, éventuellement hydroxylés, sous forme libre ou salifiée, choisis parmi l'acide propionique, l'acide citrique, l'acide tartrique, l'acide lactique, l'acide malique, l'acide succinique, l'acide glutarique, l'acide itaconique,
- les acides aminocarboxyliques sous forme libre ou salifiée choisis parmi l'acide aspartique, l'acide glutamique, la sérine, l'alanine, la déhydroalanine et leurs oligomères, l'acide iminosuccinique et ses dérivés, l'acide éthylène diamine tétraacétique,
- les monosaccharides, les oligosaccharides, les polysaccharides et leurs dérivés, choisis parmi le glucose, le galactose, le mannose, le xylose, le lyxose, le fucose, l'arabinose, le rhamnose, le ribose, le désoxyribose, le quinovose, le fructose, le sorbose, le talose, le thréose, l'érythrose, le tréhalose, le lactose, le maltose, le cellobiose, l'acide glucuronique, l'acide lactobionique, les alginates, les chitosanes et les pectines,
- l'acide ascorbique,
- l'acide phytique,
- les polymères ou copolymères d'acides carboxyliques sous forme libre ou salifiée,
- les polymères ou copolymères d'acides aminocarboxyliques sous forme libre ou salifiée choisis parmi l'acide polyaspartique et l'acide polyglutamique,
- les polymères ou copolymères de l'acide maléique ou itaconique, et
- les polymères ou copolymères de carboxyméthylinuline.

10. Procédé selon l'une quelconque des revendications 1 à 9, **caractérisé en ce que** la ou les compositions comprennent moins de 5 % en poids de sel d'aluminium, de préférence moins de 3 % en poids, de préférence moins de 1 % en poids, et de préférence sont totalement exemptes de sel d'aluminium.

11. Procédé selon l'une quelconque des revendications 1 à 10, **caractérisé en ce que** le rapport molaire entre le sel d'acide phosphate soluble dans l'eau et l'oxyde de magnésium est inférieur ou égal à 0,9, de préférence inférieur ou égal à 0,8, de préférence compris entre 0,2 et 0,9, de préférence compris entre 0,3 et 0,8.

12. Composition cosmétique prête à l'emploi, notamment pour traiter la transpiration humaine et traiter les odeurs corporelles résultant de la transpiration, qui comprend, dans un milieu cosmétiquement acceptable, de l'oxyde de magnésium et au moins un sel d'acide phosphate soluble dans l'eau qui est le dihydrogénophosphate de potassium, le rapport molaire entre le sel d'acide phosphate soluble dans l'eau et l'oxyde de magnésium étant strictement inférieur à 1.

13. Composition cosmétique selon la revendication 12, **caractérisée en ce que** le rapport molaire entre le sel d'acide phosphate soluble dans l'eau et l'oxyde de magnésium est inférieur ou égal à 0,9, de préférence inférieur ou égal à 0,8, de préférence compris entre 0,2 et 0,9, de préférence compris entre 0,3 et 0,8.
